# EUROPÄISCHE PATENTSCHRIFT

(11) **EP 4 312 898 B1**
(45) Veröffentlichungstag und Bekanntmachung des Hinweises auf die Patenterteilung: **05.03.2025**
(21) Anmeldenummer: 22719257.2
(22) Anmeldetag: 29.03.2022
(51) Int. Cl.: A61F 2/80

(54) **ORTHOPÄDISCHER SCHAFT UND VERFAHREN ZU DESSEN EINSTELLUNG**
ORTHOPEDIC SOCKET AND METHOD FOR ADJUSTING IT
DOUILLE ORTHOPÉDIQUE ET PROCÉDÉ POUR L'AJUSTER

(30) Priorität: 29.03.2021 DE 102021107816
(43) Veröffentlichungstag der Anmeldung: 07.02.2024
(73) Patentinhaber: Ottobock SE & Co. KGaA, 37115 Duderstadt (DE)
(72) Erfinder: KOPPE, Mario, 37115 Duderstadt (DE); REINELT, Stefan, 37115 Duderstadt (DE); FINKE, Lars Benjamin, 37115 Duderstadt (DE); VIER, Leonard, 37115 Duderstadt (DE); SIEGEL, Johannes, 37115 Duderstadt (DE)
(74) Vertreter: Gramm, Lins & Partner Patent- und Rechtsanwälte PartGmbB
(86) Internationale Anmeldenummer: PCT/EP2022/058254
(87) Internationale Veröffentlichungsnummer: WO 2022/207635

(56) Entgegenhaltungen:
- WO-A1-2016/091763
- WO-A1-2017/186902
- WO-A2-2008/061500
- DE-B3- 102018 129 921
- US-A1- 2010 274 364

## Beschreibung

Die Erfindung betrifft einen orthopädietechnischen Schaft, insbesondere ein Prothesenschaft, zur Anlage an und zur Aufnahme von einem Gliedmaßenstumpf oder einer Gliedmaße zumindest einer Schaftwand, eine proximalen Einstiegsöffnung und einem distalen Ende und zumindest eine Befestigungseinrichtung, die zur Festlegung zumindest einer distalen, orthopädietechnischen Komponente an dem Schaft angeordnet ist. Die Erfindung betrifft ebenfalls ein Verfahren zur Einstellung eines solchen orthopädietechnischen Schaftes.

Orthopädietechnische Schäfte, insbesondere Prothesenschäfte, dienen zur Aufnahme von Gliedmaßenstümpfen oder Gliedmaßen und können eine Vielzahl von Ausgestaltungen aufweisen. Eine Ausgestaltung eines Schaftes sieht vor, dass die Gliedmaße oder der Gliedmaßenstumpf, an den der Schaft angelegt werden soll, abgeformt wird. Gegebenenfalls mit einer Materialzugabe wird auf diese Form der Schaft aufmodelliert, beispielsweise aus einem thermoplastischen Kunststoff oder einem Faserverbundwerkstoff. Die Schaftwand ist in der Regel geschlossenwandig ausgebildet und formstabil und weist eine proximale Einstiegsöffnung auf. An dem distalen Ende oder distalen Endbereich sind Einrichtungen zur Befestigung von weiteren Komponenten, beispielsweise Gelenken oder dergleichen, angeordnet, insbesondere eingearbeitet.

Alternativ können die Schäfte in einem additiven Fertigungsverfahren hergestellt werden.

Um eine bessere Passform und eine Anpassung an unterschiedliche Konturen oder Volumina herzustellen, werden Prothesenschäfte mit einem offenen Querschnitt oder aus mehreren, relativ zueinander verlagerbaren Komponenten hergestellt. Über Motoren oder über Zugmitteln werden die Komponenten zueinander positioniert und zueinander verspannt und in der jeweiligen Position fixiert. Durch Lösen des Zugmittels oder eines Verschlusses kann das Schaftvolumen, das zur Aufnahme der Gliedmaße oder des Gliedmaßenstumpfes dient, vergrößert werden. Dies erfolgt beispielsweise beim Anlegen und Ablegen des Schaftes oder zur Entlastung der Gliedmaße oder des Gliedmaßenstumpfes, beispielsweise beim Sitzen.

Die Schäfte, insbesondere Prothesenschäfte, halten einerseits die weiteren orthopädietechnischen Komponenten sicher an der Gliedmaße oder dem Gliedmaßenstumpf und stellen darüber hinaus sicher, dass die weiteren orthopädietechnischen Komponenten korrekt zu der Gliedmaße oder dem Gliedmaßenstumpf ausgerichtet sind. Dazu ist es notwendig, dass der Orthesenschaft oder Prothesenschaft eine ausreichende Steifigkeit und Stabilität aufweist, um eine sichere Führung der daran befestigten orthopädietechnischen Komponente zu gewährleisten

Ortsfeste, rigide Anlageflächen, beispielsweise die Ramusanlage, sind eine Notwendigkeit für eine adäquate Positionierung des Schaftes an der Gliedmaße oder dem Gliedmaßenstumpf. Solche rigiden Anlageflächen sind jedoch nachteilig hinsichtlich des Komforts.

Die WO 2016/091763 A1 betrifft einen Prothesenschaft für die untere Extremität mit einem Außenschaft zum Aufnehmen eines Innenschafts, wobei der Innenschaft zum Aufnehmen eines Körpergliedstumpfs der unteren Extremität ausgestaltet ist. Der Außenschaft weist einen Umfangsabschnitt und wenigstens eine Anpassungseinrichtung aufweist, welche Teil des Außenschafts ist oder mit dem Außenschaft verbunden oder verbindbar ist. Die Anpassungseinrichtung bewirkt bei ihrer Betätigung eine Änderung der Form des Außenschafts oder eine Änderung einer Steifigkeit des Prothesenschafts oder des Außenschafts wenigstens im Bereich des Umfangsabschnitts bei unverändertem Umfang des Umfangsabschnitts.

Aufgabe der vorliegenden Erfindung ist es, einen orthopädietechnischen Schaft sowie ein Verfahren zur Einstellung eines orthopädietechnischen Schaftes bereitstellen, mit dem eine Erhöhung des Tragekomforts bei gleichzeitiger Beibehaltung einer präzisen Führung orthopädietechnischer Komponenten erreicht wird.

Erfindungsgemäß wird diese Aufgabe durch einen orthopädietechnischen Schaft mit den Merkmalen des Hauptanspruches und ein Verfahren mit den Merkmalen des nebengeordneten Anspruchs gelöst. Vorteilhafte Ausgestaltungen und Weiterbildungen der Erfindung sind in den Unteransprüchen, der Beschreibung sowie den Figuren offenbart.

Der orthopädietechnische Schaft zur Anlage an und zur Aufnahme von einem Gliedmaßenstumpf oder einer Gliedmaße mit zumindest einer Schaftwand, einer proximalen Einstiegsöffnung, einem distalen Ende und zumindest einer Befestigungseinrichtung, die zur Festlegung zumindest einer distalen orthopädietechnischen Komponente an dem Schaft angeordnet ist, sieht vor, dass an oder in der Schaftwand zumindest ein Versteifungselement angeordnet ist, dem eine Verstelleinrichtung zugeordnet ist und über das eine Steifigkeit der Schaftwand zumindest bereichsweise veränderbar, insbesondere reversibel veränderbar ist. Mit einem solchen Schaft ist es möglich, dass bestimmte Flächen oder Bereiche der Schaftwand nur dann eine notwendige Steifigkeit oder Rigidität aufweisen, wenn dies notwendig ist. Insbesondere kann bei unterschiedlichen Nutzungssituationen die Steifigkeit bereichsweise angepasst werden, um entweder eine präzise Führung und eine stabile Abstützung der Gliedmaße oder des Gliedmaßenstumpfes zu gewährleisten oder um eine ausreichende Nachgiebigkeit bereitzustellen, damit der Schaft angenehm zu tragen ist.

Das Versteifungselement wirkt in Kombination mit der Schaftwand und dient dazu, die Steifigkeit in den entsprechenden Bereichen zu verändern. Das Versteifungselement ist insbesondere eine Zusatzkomponente an einem Schaft, die daran befestigt, angeordnet, ausgebildet oder integriert ist.

Eine Ausgestaltung des Schaftes sieht vor, dass das Versteifungselement verlagerbar in oder an der Schaftwand gelagert ist, sodass eine Positionierung in unmittelbarer Nähe der in ihrer Steifigkeit zu verändernden Bereiche möglich ist. Eine Lagerung an der Schaftwand, insbesondere an der Außenseite der Schaftwand, erleichtert die Zugänglichkeit, beispielsweise um Reparaturen auszuführen oder Anpassungen vorzunehmen. Eine Anordnung in der Schaftwand nutzt die Schaftwand als solche zum Schutz und zur Bildung eines Gehäuses, sodass das Versteifungselement vor äußeren Einflüssen geschützt ist. Die Lagerung oder Anordnung innerhalb der Schaftwand ermöglicht eine kompakte Ausgestaltung und insbesondere eine glattflächige Kontur der äußeren Schaftwand.

Eine Variante sieht vor, dass dem Versteifungselement ein motorischer Antrieb zugeordnet ist, z.B. über ein Verstellelement, das zwischen dem Antrieb oder der Verstelleinrichtung und dem Versteifungselement angeordnet ist. Über den Antrieb oder die Verstelleinrichtung ist es möglich, das Verstellelement und/oder das Versteifungselement zu verdrehen, zu verschieben, zu verbiegen oder auf eine andere Art und Weise zu verlagern oder zu manipulieren, um die gewünschte Veränderung der Steifigkeit in dem jeweiligen Bereich der Schaftwand zu erreichen. Als motorischer Antrieb wird auch ein linearer Aktuator angesehen, ebenso schaltbare Magneteinrichtungen, die eine Verlagerung bewirken.

In einer Ausgestaltung ist das Verstellelement als ein Zahnrad, Rolle, Kurbel, Hebel, Zugmittel, Drehelement, Kolben, Schubmittel und/oder Ventil ausgebildet. Zugmittel sind Komponenten oder Bauteile, die Zugkräfte übertragen. Die Zugmittel können starr oder flexibel ausgebildet sein, insbesondere sind Zugmittel flexibel und unelastisch, insbesondere drucknachgiebig und zugstarr. Grundsätzlich besteht auch die Möglichkeit, dass die Zugmittel eine Elastizität aufweisen, sodass sie gleichzeitig auch als Kraftspeicher und Kraftbegrenzer dienen können. Drehelemente, die um einen Drehpunkt oder eine Drehachse verlagert werden, können beispielsweise als Räder, Rollen, Wellen, Spindeln, Schrauben oder auch verdrehbare oder verdrillbare Komponenten wie Schnüre oder Ketten sowie Stangen ausgebildet sein. Kolben werden linear bewegt und verdrängen ein Medium, in einer alternativen Ausgestaltung sind die Kolben als Drehkolben ausgebildet und verdrängen das jeweilige Medium nicht durch eine Linearbewegung, sondern durch eine Drehbewegung um eine Drehachse des Drehkolbens. Schubmittel sind Druckkräfte übertragende Komponenten, beispielsweise Stangen, Pleuel, Schubketten, oder andere druckstarre Komponenten, gegebenenfalls in Kombination mit elastischen Komponenten, die eine Kraftspeicherung und Kraftbegrenzung ermöglichen. Sofern die Verstellung der Steifigkeit über eine Zufuhr oder Abfuhr von Fluiden erfolgt, sind in einer Ausgestaltung Ventile als Schaltventile, Verstellventile oder andere verstellbare Durchflussbegrenzer ausgebildet, mit denen die Durchflussmenge eingestellt oder ein Strömungskanal gesperrt werden kann. Die Kraftspeicher sind insbesondere als Federn, komprimierbare Fluide, Speicher für elektrische Energie oder Speicher für thermische Energie ausgebildet.

Die Verstelleinrichtung ist in einer Ausgestaltung als Elektromagnet, Pumpe, Elektromotor, Heizung, Kühlelement, Piezoelement, elektroaktives Polymer, Kraftspeicher, manuell betätigbare Einrichtung oder Spannungsquelle ausgebildet. Über die Verstelleinrichtung ist es möglich, dass entsprechende Versteifungselement oder ggf. das Verstellelement zu verstellen oder zu verändern, um dadurch die Steifigkeit in dem Bereich der Schaftwand anzupassen und zu verändern. Über den Elektromagnet kann entweder ein Versteifungselement bzw. Verstellelement verlagert oder ein Magnetfeld erzeugt werden, um eine magnetorheologische Flüssigkeit hinsichtlich ihrer Viskosität zu verändern. Über eine Pumpe kann ein Fluid oder ein anderes pumpbares Medium in ein oder aus einem Volumen gepumpt werden, um die Steifigkeit des Volumens und damit des Bereiches des Prothesenschaftes, in dem sich das Volumen befindet, hinsichtlich der Steifigkeit zu verändern. Wird beispielsweise Flüssigkeit in ein Volumen hineingepumpt, vergrößert sich aufgrund des erhöhten Innendruckes in der Regel die Steifigkeit in dem Bereich, in dem sich das Volumen befindet. Die Steifigkeit kann auch erhöht werden, wenn das Volumen mit Strukturelementen befüllt ist und ein Vakuum angelegt wird. Durch den Unterdruck werden die Seitenwände gegen die Strukturelemente gedrückt und erhöhen die Steifigkeit des Schaftes in diesem Bereich. Über einen Elektromotor können mechanische Komponenten verstellt werden, beispielsweise werden Elemente mit unterschiedlichen Flächenträgheitsmomenten verdreht oder anders ausgerichtet, um eine Anpassung der Steifigkeit zu ermöglichen. Über einen Motor können auch Pumpen angetrieben, Kraftübertragungseinrichtungen verlagert oder aufgewickelt und Ventile verstellt werden. Mittels der Heizung, beispielsweise einer Widerstandsheizung, kann eine Veränderung der Viskosität und der Steifigkeit durch Erwärmen gezielter Bereiche erfolgen. Umgekehrt kann über die Kühlung bestimmter Bereiche die Festigkeit erhöht werden.

In einer Ausgestaltung ist dem Verstellelement und/oder der Verstelleinrichtung zumindest ein Versteifungselement zugeordnet, dass in oder an der Schaftwand angeordnet ist. Das Versteifungselement wird verlagert, aktiviert, in seiner Beschaffenheit verändert, in eine andere Orientierung gebracht, zu anderen Elementen in eine andere Beziehung versetzt, um dadurch eine veränderte Steifigkeit in dem jeweilige Bereich, in dem oder an dem das Versteifungselement angeordnet ist, zu bewirken. Das Versteifungselement kann beispielsweise als Fluidvolumen, Formgedächtniselement, Wellkörper, Ziehharmonikaelement, Verzahnungskörper, Feder, Schaumkörper, Phasenwechselmaterial, Klappelement, Schieber, Einsatz, Dämpfer oder Elektroadhäsionselement ausgebildet sein.

In einer Variante weist die Verstelleinrichtung einen Aktuator auf, der über eine ihm zugeordnete Steuerungseinrichtung aktivierbar und deaktivierbar ist, wobei die Steuerungseinrichtung mit zumindest einem Sensor gekoppelt ist und auf der Grundlage von Sensordaten den Aktuator aktiviert oder deaktiviert. Statt mit oder ergänzend zu einem Sensor kann die Steuerungseinrichtung auch mit einem Schalter oder einer Schalteinrichtung gekoppelt sein, über die es möglich ist, den Aktuator manuell zu aktivieren. Die Schalteinrichtung kann beispielsweise in Gestalt einer Fernbedienung oder eines Mobiltelefones oder einer mobilen Datenverarbeitungseinrichtung ausgestaltet sein, die mit der Steuerungseinrichtung koppelbar ist. Die Kopplung kann per Kabelverbindung oder drahtlos, beispielsweise über eine Funkschnittstelle, erfolgen. Dazu sind in der mobilen Datenverarbeitungseinrichtung sowie der Steuerungseinrichtung jeweils entsprechende Schnittstellen zugeordnet oder an ihnen angeordnet. Wird die Verstelleinrichtung auf der Grundlage von Sensordaten automatisch gesteuert, erfolgt eine Auswertung der Sensordaten in der Steuerungseinrichtung oder alternativ in einer externen Auswerteeinheit. Die Steuerungseinrichtung weist die notwendigen Komponenten wie Kommunikationsschnittstelle, Speicher, Prozessoren, Energiespeicher sowie gegebenenfalls Signalverstärker und integrierte Schaltungen auf. Die Steuerungseinrichtung kann mit einem oder mehreren Sensoren gekoppelt werden, wobei der Sensor oder die Sensoren aus einer Gruppe von Sensoren ausgewählt ist, die einen Temperatursensor, einen Beschleunigungssensor, eine Inertial Measurement Unit (IMU), einen Raumlagesensor, einen Neigungssensor, einen Kraftsensor, einen optischen Sensor, eine Ableiterelektrode, einen Drucksensor, einen Dehnmessstreifen und einen Widerstandssensor umfasst. Der optische Sensor dient beispielsweise zur Erfassung einer vorhandenen Sauerstoffsättigung oder zur Messung des Blutzuckerspiegels, über die Ableiterelektroden werden myoelektrische Signale erfasst. Der Widerstandsensor misst beispielsweise die Feuchtigkeit an der Innenseite der Schaftwand und kann daraus Rückschlüsse über die Belastung oder die Transpiration des Nutzers des Schaftes ziehen.

In einer Ausgestaltung ist in der Schaftwand oder an der Schaftwand eine Aufnahme für das Versteifungselement, ggf. ein Verstellelement und/oder die Verstelleinrichtung angeordnet, sodass das Verstellelement, die Verstelleinrichtung bzw. das Versteifungselement nachträglich und/oder auswechselbar an oder in der Schaftwand befestigt werden kann.

Die Schaftwand umgibt in einer Ausgestaltung die Gliedmaße oder den Gliedmaßenstumpf über dessen bzw. deren gesamten Umfang und weist somit einen geschlossenen Querschnitt auf. Die Ausgestaltung mit einem umfänglich geschlossenen Querschnitt erhöht insgesamt die strukturelle Festigkeit des Schaftes, über die Bereiche mit der einstellbaren Steifigkeit können die Anforderungen an den Komfort entsprechend angepasst werden.

Das Versteifungselement und die Verstelleinrichtung oder das Verstellelement, die Verstelleinrichtung und das Versteifungselement sind in einer Ausgestaltung als ein Versteifungsmodul zusammengefasst. Dieses Versteifungsmodul kann auswechselbar an oder in der Schaftwand angeordnet werden, um einen Austausch vorhandener Komponenten, eine Nachrüstung oder eine Anpassung an den jeweiligen Nutzer vornehmen zu können. Das Versteifungsmodul kann industriell vorgefertigt und hinsichtlich der einzuhaltenden Sicherheitsbestimmungen, der Wasserdichtigkeit und anderer Anforderungen vorab getestet werden. Das Versteifungsmodul kann reversibel abnehmbar an der Schaftwand festgelegt werden, beispielsweise formschlüssig oder kraftschlüssig.

Bei dem Schaft besteht die Möglichkeit, industriell vorgefertigte, modulare Komponenten in individuelle Prothesenschäfte oder Orthesenschäfte zu integrieren, wobei sich diese Komponenten in ihrer Steifigkeit reversibel einstellen lassen, wodurch die Steifigkeit des Schaftes beziehungsweise der Schaftwand an besonderen Stellen oder in besonderen Bereichen, die individuell auswählbar sind, anpassbar sind. Für Orthesen wird der Begriff Schaft auch für Schienen, Schalen, Korsetts und/oder einen Beckenkorb verwendet. Aufgrund der Möglichkeiten, einzelne Anlageflächen in dem Schaft reversibel hinsichtlich der Steifigkeit verändern zu können, kann die Gesamtcharakteristik des Schaftes situationsbedingt angepasst werden. Es ist mit einem solchen Schaft nicht mehr notwendig, einen Kompromiss zwischen Komfort und Funktion einzugehen, der für alle Nutzungssituationen passend ist und damit in der Regel in keiner Situation die optimale Funktionalität bietet.

In einer Ausgestaltung sind in einem proximalen Randbereich der Schaftwand Sektoren mit unterschiedlicher radialer Elastizität ausgebildet. Der Schaft, insbesondere in einer Ausgestaltung als Prothesenschaft, umgibt den Stumpf oder die Gliedmaße im Wesentlichen über den gesamten Umfang. Über den Umfang gesehen kann ein Schaft in unterschiedliche Sektoren eingeteilt sein, die unterschiedliche Aufgaben übernehmen und die bei unterschiedlichen Tätigkeiten oder Zuständen unterschiedlichen Belastungen ausgesetzt sind. Bei einem Oberschenkelschaft ist dies beispielsweise ein lateraler Sektor, der insbesondere für die seitliche Führung und Stabilität bei Belastungen in Richtung auf die Körpermitte ausschlaggebend ist. Der anteriore Sektor dient zur Abstützung in die Richtung oder Belastungsrichtung des vorderen Bereiches, der posteriore Sektor zur Abstützung in dem gegenüberliegenden, hinteren Bereich. Ein medialer Sektor nimmt Belastungen auf, die den Schaft von der Körpermitte weg bewegen sollen. Bei einem Oberschenkelschaft kommt die sogenannte Ramusanlage in dem Bereich des Sitzbeines hinzu. Bei anderen Schaftarten sind insbesondere nur die ersten vier genannten Sektoren vorhanden, grundsätzlich können auch mehr oder weniger Sektoren an dem Schaft angeordnet oder ausgebildet sein. Aufgrund der unterschiedlichen Belastungen, Belastungsarten, Belastungsrichtungen, Belastungsgrößen und auch Belastungshäufigkeiten ist es vorteilhaft, wenn die Sektoren mit in radialer Richtung unterschiedlicher Elastizität ausgebildet sind, sodass unterschiedlich steife Schaftwandabschnitte in dem proximalen Randbereich, der einen oberen Abschluss des Prothesenschaftes bildet und ungefähr bis zur oberen Hälfte oder bis zum oberen Drittel der Gesamtlänge des Schaftes reichen kann, bereitgestellt werden können. Über die Verstelleinrichtung und gegebenenfalls ein Verstellelement können die Steifigkeiten in den einzelnen Sektoren separat und unabhängig voneinander in beliebigen Kombinationen und in notwendigen Größen eingestellt oder verändert werden.

Die Sektoren können unterschiedliche Materialien, unterschiedliche Materialstärken, unterschiedlich breite Ausnehmungen und/oder unterschiedliche Dichten an Ausnehmungen aufweisen. Werden beispielsweise die Versteifungselemente vollständig deaktiviert oder auf den jeweils minimalen Widerstand eingestellt, können sich unterschiedliche Grundsteifigkeiten oder Grundwiderstände gegen eine radiale Verlagerung des Schaftes aufgrund der grundsätzlichen Konstruktion in dem proximalen Randbereich ergeben. Die unterschiedlichen Grundsteifigkeiten können durch unterschiedliche Materialien in den unterschiedlichen Sektoren bereitgestellt werden. Sofern gleiche Materialien verwendet werden, beispielsweise ein Kunststoff oder ein faserverstärkter Kunststoff, können unterschiedliche Materialstärken zu unterschiedlichen Steifigkeiten in den jeweiligen Sektoren führen. Ergänzend oder alternativ zu den beiden vorgenannten Maßnahmen können Ausnehmungen innerhalb der Sektoren vorgesehen sein, die die Steifigkeit des jeweiligen Abschnittes der Schaftwand beeinflussen. Eine Möglichkeit zur Ausgestaltung der Ausnehmungen sind Schlitze, die in proximal-distal-Richtung eingebracht sind. Die Schlitze können bis zum oberen Abschluss des Prothesenschaftes reichen und erstrecken sich in distaler Richtung gegebenenfalls bis zu dem Ende des proximalen Randbereiches. Die Breite der Ausnehmungen oder Schlitze kann zwischen den Sektoren oder auch innerhalb eines Sektors variieren. Ebenso kann die Dichte der Ausnehmungen der einzelnen Sektoren verschieden sein. Je höher die Anzahl der Ausnehmungen auf einer normierten Umfangslänge ist, desto geringer ist die Steifigkeit, je höher die Länge der Ausnehmungen ist, desto geringer ist die Steifigkeit und je größer die Breite der Ausnehmungen ist, desto geringer ist die Steifigkeit in dem jeweiligen Segment.

In einer Ausgestaltung sind den einzelnen Sektoren verlagerbare Schaftwandsegmente als Versteifungselemente zugeordnet, die einzeln oder auch in Kombination miteinander aktuiert oder verlagert werden, um unterschiedliche Steifigkeiten in dem proximalen Randbereich bereitzustellen.

In einer Ausgestaltung ist in der Schaftwand zumindest ein flexibler Bereich ausgebildet, der mit einem Versteifungselement gekoppelt ist. Der flexible Bereich lässt beispielsweise immer eine Verlagerung des Materials innerhalb des flexiblen Bereiches radial nach innen in Richtung auf den Stumpf oder die Gliedmaße zu. Ist das Versteifungselement entspannt oder nicht aktiviert, lässt sich das Material des flexiblen Bereiches auch in die entgegengesetzte Richtung bewegen, sodass ein vergrößertes Spiel oder eine vergrößerte Beweglichkeit der Gliedmaße oder des Stumpfes in diesem Sektor erreicht werden kann. Das Versteifungselement ist beispielsweise ein Zugmittel wie ein Gurt oder ein Seil, das gespannt wird, sodass eine Verlagerung des Materials radial nach außen, beispielsweise eines Schaumstoffes oder eines Textils, nicht oder nur in einem beschränkten Maß möglich ist. Eine nicht vorhandene oder verringerte Nachgiebigkeit radial nach außen ist bei hohen Belastungen, beispielsweise bei der schnellen Ausführung von Tätigkeiten, vorteilhaft. Umgekehrt ist eine Entspannung des Zugmittels beispielsweise bei einer Inaktivität vorteilhaft, wenn keine Belastungen aufgenommen werden müssen und nur sichergestellt sein muss, dass sich der Schaft nicht von der Gliedmaße oder dem Stumpf des Nutzers löst.

Das Verfahren zur Einstellung eines orthopädietechnischen Schaftes zu Anlage und Aufnahme von einem Gliedmaßenstumpf oder einer Gliedmaße, wobei ein Schaft mit zumindest einer Schaftwand bereitgestellt wird und der Schaft eine proximale Einstiegsöffnung, ein distales Ende und zumindest eine Befestigungseinrichtung aufweist, die zur Festlegung zumindest einer distalen orthopädietechnischen Komponente an dem Schaft angeordnet ist, sieht vor, dass die Steifigkeit der Schaftwand über zumindest eine Verstelleinrichtung und ein Versteifungselement zumindest bereichsweise verändert wird. Die situationsbedingte oder frei gewählte, reversible Veränderbarkeit der Steifigkeit in bestimmten Bereichen hat zur Folge, dass der Schaft als solches in seinen mechanischen Eigenschaften eingestellt werden kann und dass durch die situationsbedingte Einstellbarkeit eine optimale Funktionalität bereitgestellt wird.

Die Veränderung der Steifigkeit erfolgt in einer Ausgestaltung durch eine Verlagerung eines Verstellelementes, eine Verstellung, Verlagerung oder Veränderung eines Versteifungselementes, das an oder in der Schaftwand angeordnet ist und/oder durch Aktivierung oder Deaktivierung der Verstelleinrichtung. Die Veränderung des Versteifungselementes erfolgt beispielsweise durch eine Verschiebung, eine Stauchung, eine Veränderung eines Anstellwinkels eines Abstützelementes oder das Einführen oder Entfernen von Material in Zwischenräumen zwischen Abschnitten, die durch das Versteifungselement ausgebildet werden.

In einer Variante kann die Veränderung der Steifigkeit durch das Verdrehen eines Federelementes erfolgen, wobei das Federelement je nach Orientierung und Ausrichtung unterschiedliche Widerstände gegen eine Verformung bereitstellt.

Alternativ oder ergänzend zu einem Verdrehen eines Federelementes kann dies auch verschoben werden, beispielsweise in einen Bereich, der grundsätzlich zur Veränderung der Steifigkeit vorgesehen ist, hinein oder aus diesem hinaus. Wenn ein Federelement in den Bereich hineingeschoben wird, erhöht sich die Steifigkeit, wird es herausbewegt, verringert sich die Steifigkeit der Schaftwand in diesem Bereich. Sofern das Federelement permanent vorhanden ist und nicht aus dem jeweiligen Bereich entfernt werden soll, kann das Federelement auch blockiert werden, sodass die durch das Federelement grundsätzlich bereitgestellte Nachgiebigkeit nicht mehr zur Verfügung steht und dadurch die Schaftwand in diesem Bereich versteift wird. Das Versteifungselement oder das Verstellelement kann verlagert werden, beispielsweise verdreht, hineingeschoben, herausgezogen oder komprimiert. Sofern ein Volumen in dem Bereich der veränderbaren Steifigkeit vorhanden ist, kann durch Befüllen oder Entleeren dieses Volumens mit einem entsprechenden Fluid und/oder eine Veränderung der Viskosität eines Mediums, das in dem Volumen vorhanden ist, die Steifigkeit verändert werden. Bereiche können erwärmt oder abgekühlt werden, um durch Veränderung der temperaturabhängigen Biegesteifigkeit eine gewünschte Veränderung zu erreichen. Versteifungselemente können formschlüssig miteinander in Eingriff gebracht werden, beispielsweise indem zueinander korrespondierende Verzahnungen in und außer Eingriff miteinander gebracht werden.

Die Änderung der Steifigkeit kann auf der Grundlage von Sensordaten erfolgen, die von zumindest einem Sensor ermittelt und einer Steuerungseinrichtung übermittelt werden, die wiederum einen Aktuator aktiviert oder deaktiviert. Über den Aktuator werden dann die jeweils notwendigen Aktionen eingeleitet, um die Steifigkeit zu verändern, beispielsweise eine Heizung eingeschaltet, eine Kühlung aktiviert, ein Versteifungselement verdreht oder verlagert oder eine andere Maßnahme ergriffen, um den jeweils gewünschten Bereich mit der jeweils gewünschten Steifigkeit bereitzustellen. Insbesondere wird die Steifigkeit durch Verdrehen, Verschieben, Spannen, Blockieren und/oder Aktivieren eines Verstellelementes bzw. der Versteifungselemente verändert.

In einer Weiterbildung weist die Schaftwand Sektoren auf, die um den Umfang verteilt sind, die unabhängig voneinander hinsichtlich ihrer Steifigkeit verändert werden. Die Veränderung der Steifigkeit erfolgt in einer Ausgestaltung während der Benutzung des orthopädietechnischen Schaftes automatisch, vorteilhafterweise in Echtzeit und/oder in Abhängigkeit von der Bewegungssituation, in der sich der Nutzer befindet, beispielsweise der Gangsituation, wenn die orthopädietechnische Einrichtung für die unteren Gliedmaßen ausgebildet ist, insbesondere als Prothesenbein. Dabei werden die Daten von Sensoren aufgenommen, einer Auswerte- und Steuerungseinrichtung übermittelt und auf der Grundlage dieser Sensordaten und der Auswertung wird die Verstelleinrichtung aktiviert, deaktiviert oder moduliert, um den jeweils gewünschten oder erforderlichen Grad an Steifigkeit einzustellen. Dadurch ist es möglich, an die jeweilige Situation angepasste Steifigkeiten einzustellen, insbesondere aufgeteilt nach Sektoren, für die in dem proximalen Randbereich eine Anpassung an die Notwendigkeiten oder Vorlieben des jeweiligen Nutzers erfolgt. Die Sensordaten können von Sensoren stammen, die an dem Schaft angeordnet und/oder an einer Komponente angeordnet oder dieser zugeordnet sind, die an dem Schaft befestigt ist. Ist beispielsweise eine Widerstandseinrichtung Teil der orthopädietechnischen Einrichtung, an der der orthopädietechnische Schaft angeordnet ist, können die Sensoren, die für die Steuerung der Widerstandseinrichtung verwendet werden, auch zur Erkennung von Belastungssituationen eingesetzt werden. Auf der Grundlage der jeweiligen Belastungssituationen kann dann nicht nur der Widerstand eingestellt, sondern auch die Steifigkeit in dem jeweiligen Sektor des Schaftes verändert werden.

Nachfolgend werden Ausführungsbeispiele der Erfindung anhand der beigefügten Figuren näher erläutert. Es zeigen:
Figur 1 - eine Gesamtdarstellung eines Prothesenbeines;
Figur 2 - einen Prothesenschaft in Einzeldarstellung;
Figur 3 - eine schematische Darstellung eines Schnittes durch eine Schaftwand;
Figur 4 - eine Draufsicht auf einen Prothesenschaft mit einem verschieblichen Versteifungselement;
Figur 5 - zwei Darstellungen eines Schaftrandabschnittes in unterschiedlichen Steifigkeitszuständen; Figuren 6a und 6b - Darstellungen eines Prothesenschaftabschnittes mit einem verschieblichen Versteifungselement;
Figuren 7a und 7b - Darstellungen eines Schaftwandabschnittes mit verdrehbaren Versteifungselementen;
Figur 8 - eine Schnittdarstellung eines elastischen Schaftwandabschnittes mit einem parallelen Dämpferelement;
Figur 9a und 9b - Darstellungen eines Schaftwandabschnittes mit befüllbaren und entleerbaren Volumen;
Figur 10 - zwei Schnittdarstellungen mit zwei Versteifungselementen;
Figur 11 - in Längserstreckung verlagerbare Versteifungselemente,
Figur 12 - eine schematische Darstellung mit einem kammartigen Versteifungselement;
Figur 13 - eine Darstellung mit einem horizontal verschieblichen Versteifungselement;
Figur 14 - eine Variante der Figur 13;
Figur 15 - eine Schnittdarstellung durch einen Schaft mit magnetorheologischer Flüssigkeit;
Figur 16 - eine Schnittdarstellung durch eine Schaftwand mit einem hydraulischen Versteifungselement;
Figuren 17a und 17b - zwei Darstellungen mit einem Versteifungselement in Zieharmonika-Ausgestaltung;
Figur 18 - eine Darstellung klappbarer Versteifungselemente in unterschiedlichen Positionen;
Figur 19 - ein Prothesenschaft mit einem verlagerten Versteifungselement;
Figur 20 - ein Schaftwandabschnitt mit einem spannbaren Versteifungselement;
Figur 21 - eine Ausgestaltung eines drehbaren Versteifungselementes;
Figur 22 - eine Variante der Figur 20;
Figur 23 - eine Ausgestaltung eines Schaftes mit einem viskoelastischen Element mit einem magnetorheologischen Fluid
Figur 24 - eine Variante der Figur 2 mit Verstelleinrichtung;
Figur 25 - eine Variante der Figur 6;
Figur 26 - eine Variante der Figur 25;
Figur 27 - eine Ausgestaltung eines Versteifungsmoduls;
Figur 28 - eine Darstellung einer Führung für Versteifungselemente;
Figur 29 - eine Darstellung eines Prothesenschaftes;
Figur 30 - eine Draufsicht auf einen Prothesenschaft;
Figur 31 - mögliche Schaltungen;
Figur 32 - Situationen mit unterschiedlichen Steifigkeiten;
Figur 33 - eine Steuerung der Verstellung der Steifigkeiten;
Figur 34 - eine Darstellung eines Prothesenschaftes;
Figur 35 - ein Einzeldarstellung einer Verstelleinrichtung;
Figur 36 - Einzeldarstellungen von Komponenten einer Verstelleinrichtung;
Figur 37 - eine Seitansicht einer Variante eines Prothesenschaftes;
Figur 38 - eine Variante eines Prothesenschaftes mit abnehmbaren Versteifungselementen;
Figur 39 - eine Variante eines Versteifungselementes mit Verstelleinrichtung undVerstellelement;
Figur 40 - eine Variante der Figur 39;
Figur 41 - ein Versteifungselement als Kniehebelverschluss;
Figur 42 - eine Draufsicht auf eine Variante eines Prothesenschaftes; sowie
Figur 43 - zwei Ansichten einer weiteren Variante.

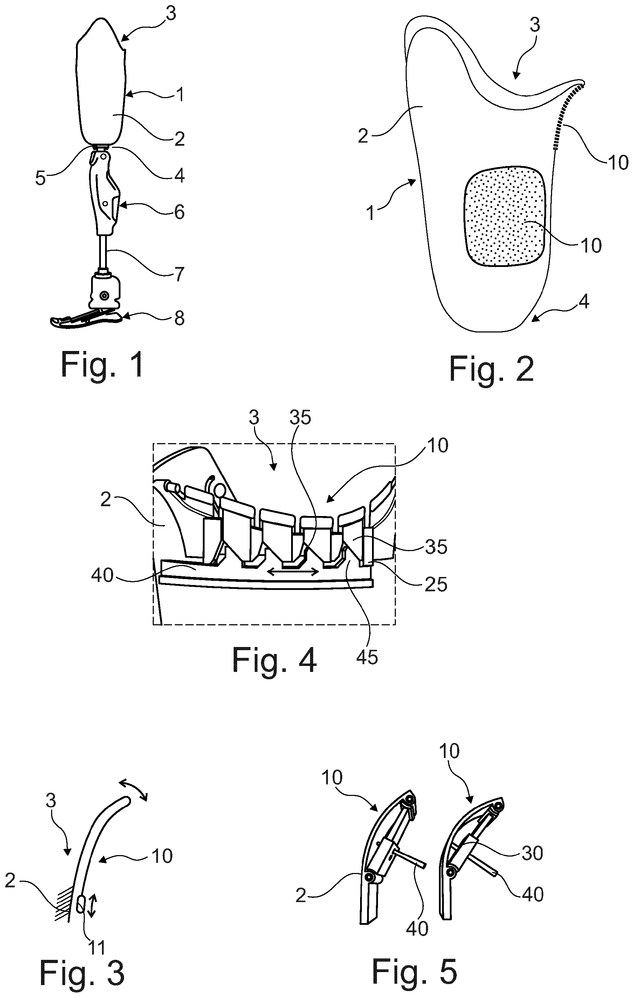

In der Figur 1 ist ein Schaft 1 in Gestalt eines Prothesenschaftes mit einer Befestigungseinrichtung 5 in dem distalen Endbereich gezeigt. Die Befestigungseinrichtung 5 ist als sogenannter Pyramidenadapter ausgebildet und dient zur Befestigung eines Prothesenkniegelenkes 6, das über ein Unterschenkelrohr 7 mit einem Prothesenfuß 8 gekoppelt ist.

In der Figur 2 ist in einer schematischen Darstellung ein Schaft 1 in Gestalt eines Prothesenschaftes mit einer Schaftwand 2, einer proximalen Einstiegsöffnung 3 und in einem distalen Endbereich 4 dargestellt. Der distale Endbereich 4 ist in dem dargestellten Ausführungsbeispiel geschlossen und bildet eine Endkappe aus, an dessen distalem Ende eine nicht dargestellte Befestigungseinrichtung für ein Gelenk, beispielsweise ein Prothesenkniegelenk oder ein Prothesenknöchelgelenk angeordnet ist. Die Schaftwand besteht überwiegend aus einem formstabilen Material und ist mit einem geschlossenen Querschnitt ausgebildet, sodass ein innerhalb des von der Schaftwand 2 umgebenen Volumens ein Gliedmaßenstumpf aufgenommen werden kann. Der Gliedmaßenstumpf kann entweder unmittelbar in den Prothesenschaft 1 und eingeführt und darin festgelegt werden oder mittels einer sogenannten Linertechnologie, bei der ein flexibler und elastischer Prothesenliner mittels Vakuumtechnologie oder einer mechanischen Verriegelung mittels eines Pinlock verriegelt wird.

Auf der lateralen Seite ist der Prothesenschaft 1 mit einer Erhöhung der Schaftwand 2 ausgebildet, um eine sichere Führung zu ermöglichen, auf der medialen Seite ist die Schaftwand 2 tiefer ausgeschnitten, um die sogenannte Ramusanlage auszubilden. In einem mittleren Bereich der Schaftwand 2 ist ein erster Bereich 10 ausgebildet, in dem die Steifigkeit der Schaftwand 2 veränderlich ist, beispielsweise über ein befüllbares und entleerbares Volumen, das sich in der dort hohl ausgebildeten Schaftwand 2 befindet. Darüber hinaus ist in dem Bereich der Ramusanlage ein zweiter Bereich 10 in der Schaftwand ausgebildet, der in seiner Steifigkeit veränderbar ist. Möglichkeiten der Verstellung der Steifigkeit in den dargestellten Bereichen 10 sowie in anderen Bereichen werden nachfolgend näher erläutert.

Figur 3 zeigt eine erste Ausführungsform in Gestalt einer schematischen Schnittdarstellung durch eine Schaftwand 2 eines Schaftes mit dem proximalen Rand in dem Gebiet der Ramusanlage. Die Schaftwand 2 weist in diesem Bereich eine Doppelfeder auf, deren Fußpunkt 11 verschieblich gelagert ist. Die Verschiebung des Fußpunktes 11 kann motorisch oder über Magnete verlagert werden. Auch eine pneumatische oder hydraulische Verlagerung des Fußpunktes 11 ist in einer Ausgestaltung vorgesehen. Wird der Fußpunkt 11 nach unten verlagert, wird die Doppelfeder in dem Bereich 10 vorgespannt und die Steifigkeit in dem Bereich 10 verändert sich. Das Versteifungselement ist dabei die Doppelfeder, die über einen nicht dargestellten Aktuator als Verstelleinrichtung oder durch manuelle Verstellung über einen Hebel oder Schieber als Verstelleinrichtung in ihrer Steifigkeit durch eine Veränderung der Position des Fußpunktes 11 beeinflusst werden kann.

Eine Variante der Verstellung der Steifigkeit in der Ramusanlage ist in der Figur 4 gezeigt, die eine Draufsicht auf den proximalen Randbereich des Schaftes mit der Schaftwand 2 und der proximalen Einstiegsöffnung 3 zeigt. Der proximale Schaftrand ist geschlitzt ausgebildet und weist Schrägen 35 auf, die mit einem Versteifungselement 40 in Eingriff stehen. Wird das Versteifungselement 40 nach rechts verschoben, beispielsweise über einen Seilzug, der aufgewickelt wird oder über einen Zahnradantrieb, werden die Schrägen 45 des Versteifungselementes 40 gegen die Schrägen 35 an der Schaftwand 2 gedrückt und damit nach oben geschoben. Gleichzeitig werden die Keile gegen die Widerlager 25 aufgrund der in Umfangsrichtung wirkenden Kraftkomponente gepresst, wodurch eine Erhöhung der Steifigkeit in diesem Bereich 10 stattfindet. Wird das Versteifungselement 40 nach links verlagert, wird die Nachgiebigkeit in diesem Bereich vergrößert.

Figur 5 zeigt eine Variante einer Schaftwand 2 mit einer verstellbaren Steifigkeit durch Verdrehen des Versteifungselementes 40, beispielsweise über einen Motor als Verstelleinrichtung 30. Das Versteifungselement 40 wird über die Verstelleinrichtung 30, die an der Schaftwand 2 gelagert ist, in Richtung auf die Schaftwand 2 bewegt. Die Verstelleinrichtung 30 ist verschwenkbar nach proximal und distal an der Schaftwand 2 gelagert ist und bewirkt dadurch, dass sich die Schaftwand 2 in dem Bereich stärker krümmt und eine höhere Vorspannung und damit eine höhere Steifigkeit aufweist. Das Versteifungselement 40 kann auch nachgiebig ausgebildet sein und durch eine Veränderung des Anpressdruckes über die Verstelleinrichtung 30 punktuell oder flächig in dem Bereich 10 eine Veränderung der Steifigkeit in der Schaftwand 2 herbeiführen. Die Verlagerung des Versteifungselementes 40 durch die Stelleinrichtung 30 kann beispielsweise durch einen Gewindeantrieb, eine Spindel, einen Motor oder einen Hebel erfolgen, die Fixierung in der gewünschten Position erfolgt beispielsweise aufgrund einer Selbsthemmung eines Gewindes oder durch das Klemmen des Versteifungselementes 40 in der gewünschten Position. Das Versteifungselement 40 wirkt dabei auf die Schaftwand 2 ein.

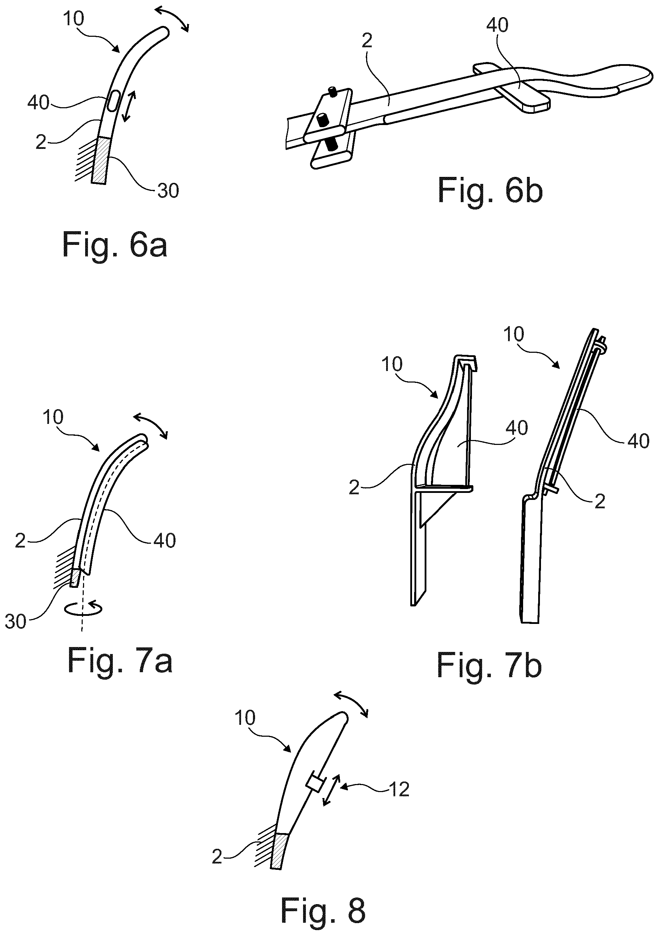

Figuren 6a und 6b zeigen eine Schaftwand 2 mit einem verschieblich gelagerten Versteifungselement 40. Die Schaftwand 2 ist in dem Bereich 10 mit der einstellbaren Steifigkeit in Gestalt einer Doppelfeder ausgebildet und weist ein verschieblich gelagertes Versteifungselement 40 als Zwischenelement auf, das zur Verblockung der Einzellagen der Doppelfeder dient. Je nach Positionierung und Orientierung des Versteifungselementes 40 wird eine veränderte Steifigkeit in diesem Bereich erzielt. Die Verstellung des Versteifungselementes 40 kann motorisch über eine Verstelleinrichtung 30 oder manuell erfolgen.

Die Figuren 7a und 7b zeigen eine Ausgestaltung der Schaftwand 2 in einem proximalen Endbereich mit einer Doppelfeder mit einer rotatorisch verstellbaren Einzellage als Versteifungselement 40. In der Figur 6b sind unterschiedliche Positionen des Versteifungselementes 40 gezeigt. In der rechten Darstellung der Figur 6b ist eine verringerte Steifigkeit im Vergleich zu der linken Darstellung gegeben. In der linken Darstellung ist die nicht rotationssymmetrische Formgestaltung des Versteifungselementes 40 zu erkennen, wodurch sich eine spezielle Form des Schaftrandes im verdrehten Zustand ergibt. Auch hier bewirken unterschiedliche Stellungen des Versteifungselementes 40 unterschiedliche Steifigkeiten der Schaftwand 2

Figur 8 zeigt die Ausgestaltung der Schaftwand 2 mit dem in der Steifigkeit verstellbaren Bereich 10 in Gestalt einer Einzelfeder, an der ein Dämpferelement 12 angeordnet ist. Der Wandbereich 10 führt eine Verschwenkung in dem proximalen Endbereich aus, die durch eine in Längserstreckung wirkende Verlagerung des Dämpferelementes beeinflusst wird. Je höher der Widerstand in dem Dämpferelement 12 ist, desto höher sind die Steifigkeit der Feder und damit der Bereich 10 der Schaftwand 2. Das Dämpferelement 12 kann als Hydraulikdämpfer, Pneumatikdämpfer oder Festkörperdämpfer ausgebildet sein und ist einstellbar, beispielsweise über ein Vorspannelement oder eine Verstelleinrichtung, über die ein Ventil verlagert wird. Über die Vorspanneinrichtung kann ein Festkörperdämpfer komprimiert oder entspannt werden, wodurch sich der Verformungswiderstand vergrößern oder verkleinern lässt. Das Dämpferelement 12 ist in dieser Ausgestaltungsform das Versteifungselement, das die Veränderung der Steifigkeit der Schaftwand 2 bewirkt.

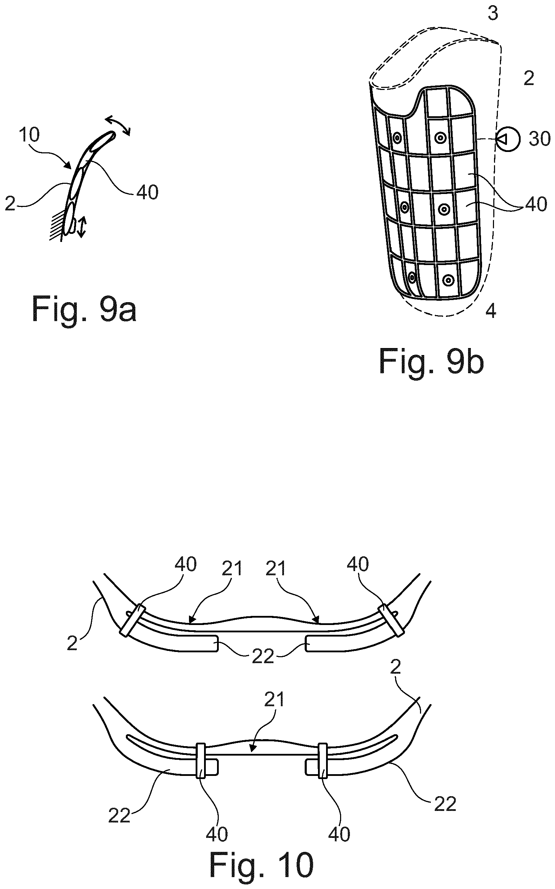

In den Figuren 9a und 9b ist eine Variante mit befüllbaren Volumen gezeigt. Die Schaftwand 2 ist doppelwandig ausgebildet, insbesondere in Gestalt einer elastischen Doppelfeder, und weist innerhalb des Hohlraumes zwischen den beiden Wänden Versteifungselemente 40 auf, die über eine Pumpe als Verstelleinrichtung 30 befüllbar oder entleerbar sind. Je nach Befüllungsgrad verändert sich die Steifigkeit in dem jeweiligen Bereich. Die Pumpe kann als Hydraulikpumpe oder als Pneumatikpumpe ausgebildet sein. Die Verstelleinrichtung 30 ist manuell oder automatisch aktivierbar oder deaktivierbar. Eine automatische Aktivierung kann beispielsweise auf Grundlage von Sensordaten erfolgen, beispielsweise Raumlagedaten, Druckdaten oder Temperaturdaten. Die Sensordaten werden einer Steuerungseinrichtung zur Verfügung gestellt, die die Daten auswertet und einen Aktivierungs- oder Deaktivierungsbefehl dem jeweiligen Aktuator übermittelt.

In der Figur 10 ist eine Detailansicht einer Schnittdarstellung in Draufsicht gezeigt, bei der die Schaftwand 2 in unterschiedliche Schaftwandbereiche eingeteilt ist. Ein innerer, durchgängiger Schaftwandbereich 21 erstreckt sich um den Umfang einer nicht dargestellten Gliedmaße oder eines Gliedmaßenstumpfes. Parallel dazu sind auf der Außenseite des durchgängigen, inneren Schaftwandbereiches 21 zwei äußere Schaftwandbereiche 22 angeordnet, in dem dargestellten Ausführungsbeispiel angeformt. Diese äußeren Schaftwandbereiche 22 erstrecken sich über eine gewisse Höhe oder eine gewisse Proximal-Distal-Erstreckung entlang der Außenseite des Prothesenschaftes an der Schaftwand. Eine oder mehrere, verschiebliche Versteifungselemente 40 sind ebenfalls an der Schaftwand 2 angeordnet und sind in dem dargestellten Ausführungsbeispiel als Klammer oder Schieber ausgebildet, die auf der Innenseite des inneren Schaftwandbereiches 21 und auf der Außenseite des äußeren Schaftwandbereiches 22 angeordnet sind. Über die Versteifungselemente 40 wird eine veränderliche Zuordnung der inneren und äußeren Schaftwandbereiche 21, 22 vorgenommen. Die Versteifungselemente 40 können auch in dem Zwischenraum zwischen den äußeren und inneren Schaftwandbereichen 21, 22 und/oder in Nuten oder Führungen in den Schaftwandbereichen 21, 22 angeordnet sein. Die beiden äußeren Schaftwandbereiche 22 bilden zwischen sich eine Lücke aus, sodass dazwischen lediglich der innere Schaftwandbereich 21 als Gesamtmaterialstärke der Schaftwand 2 in Radialerstreckung zur Verfügung steht. In der oberen Darstellung der Figur 10 sind die Versteifungselemente 40 maximal voneinander weg verlagert, sodass eine mechanische Zuordnung und Kraftübertragung zwischen dem äußeren Schaftwandbereich 22 und dem inneren Schaftwandbereich 21 in dem Bereich der Aufspaltung der beiden Schaftwandbereiche 21, 22 erfolgt. Dadurch wird der Anteil des inneren Schaftwandbereiches 21, der alleine zur Bereitstellung einer Stabilität oder Steifigkeit der Schaftwand 2 beiträgt, vergrößert. In der unteren Darstellung sind die beiden Versteifungselemente 40 aufeinander zu verlagert, sodass größere Bereiche der äußeren Schaftwandbereiche 22 bei einer Belastung der Schaftwand 2 zur Aufnahme von Kräften beitragen und somit eine Stabilitätserhöhung und eine Vergrößerung der Steifigkeit in diesem Bereich eingestellt wird. Es können mehrere, in Proximal-Distal-Richtung beabstandete Versteifungselemente 40 an der Schaftwand 2 vorgesehen sein, um auch in Proximal-Distal-Richtung unterschiedliche Steifigkeiten über eine unterschiedliche Positionierung der einzelnen Versteifungselemente 40 bereitzustellen. Die Verstellung der Versteifungselemente 40 kann manuell durch Verschieben erfolgen, alternativ können Antriebe einer Verstelleinrichtung den Versteifungselementen 40 zugeordnet sein, beispielsweise Magnete, Motoren, Pumpen oder ähnliches, um eine Verlagerung entlang eines Verschiebeweges zu erreichen. Alternativ zu einer Verschiebung von klammerartigen oder schieberartigen Versteifungselementen 40 können diese auch als Klemmen ausgebildet sein, die den äußeren Schaftwandbereich 22 an den inneren Schaftwandbereich 21 heranziehen oder herandrücken um dadurch eine Verdickung und Vergrößerung der effektiven Materialstärke in diesem Bereich zu begründen. Alternativ zu einer umfänglichen Verlagerung der Versteifungselemente 40 können diese auch in Proximal-Distal-Richtung verlagerbar ausgestaltet sein. Die Schaftwandbereiche 21, 22 sind beispielsweise elastisch ausgebildet, sodass durch Verschieben der Versteifungselemente 40 nach oben und unten entlang der Längserstreckung des Schaftes 1 die Steifigkeit in diesem Bereich verändert werden kann.

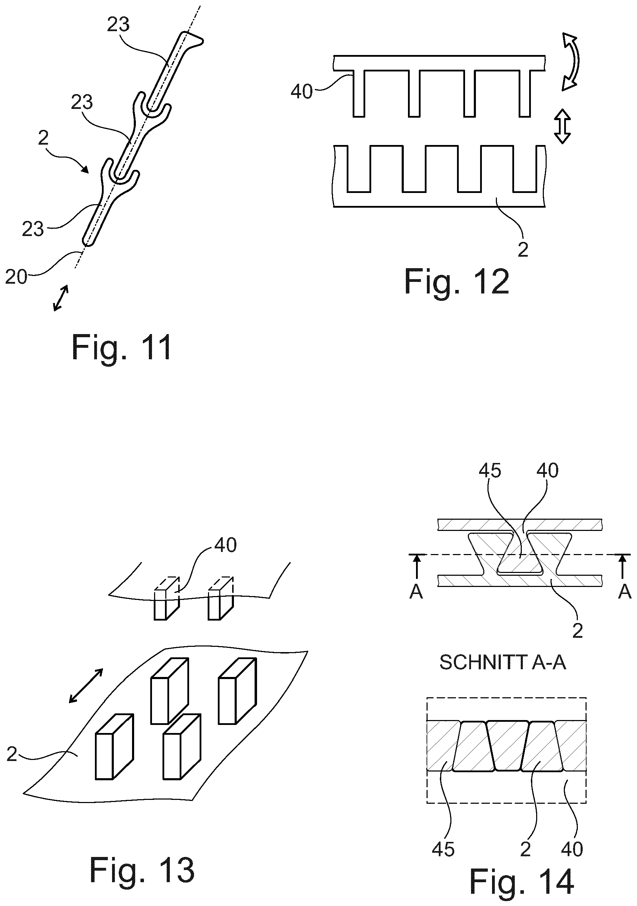

Figur 11 zeigt eine Variante, bei der Schaftwandsegmente 23 in ihrer Längserstreckung gegeneinander verschiebbar sind. Die jeweiligen Schaftwandsegmente 23 sind klappbar oder verschwenkbar zueinander aneinander gelagert und weisen entsprechende Lagerstellen auf, die in dem dargestellten Ausführungsbeispiel klammerartig ausgebildet sind. Die korrespondierend geformten Bereiche des Schaftwandsegmentes 23 werden in diese Klammer hineingezogen oder darauf herausgedrückt, wodurch sich eine Verkeilung der Schaftwandsegmente 23 ergibt. Die Verkeilung kann beispielsweise über ein Zugmittel oder eine Zug-Druck-Stange als Verstellelement 20 erfolgen, das entlang der Längserstreckung der Schaftwandsegmente 23 verlagerbar ist. Werden die Schaftwandsegmente 23 aufeinander zu verlagert und miteinander verkeilt, erhöht sich die Steifigkeit, wird eine umgekehrte Bewegung ausgeführt oder zugelassen, beispielsweise indem ein Zugmittel entgegen einer Federkraft entspannt wird und die Federkraft oder ein elastisches Element die Schaftwandsegmente 23 auseinanderdrückt, wird eine erhöhte oder erleichterte Verschwenkbarkeit der Schaftwandsegmente 23 zueinander zugelassen und dadurch die Steifigkeit der Schaftwand 2 bereichsweise verändert.

In der Figur 12 ist eine weitere Variante gezeigt, bei der an der Schaftwand 2 kammartige Vorsprünge bzw. Einschnitte an der Außenseite angeordnet sind. In die Freiräume zwischen den Zinken oder Vorsprüngen kann über ein Versteifungselement 40, das in Richtung auf die Schaftwand 2 bewegt oder davon entfernt werden kann, die Steifigkeit und Stabilität der Schaftwand 2 verändert werden. An dem Versteifungselement 40 sind korrespondierende Freiräume und Zinken angeordnet, die in die Freiräume zwischen den Zinken an der Schaftwand 2 eingeführt oder aus diesen entfernt werden können. Je nachdem, wie weit die Versteifungselemente 40 oder Zinken in die Freiräume eingeführt werden, wird die Steifigkeit in diesem Bereich vergrößert oder verringert.

Eine Variante der Figur 12 ist in der Figur 13 gezeigt, bei der das Versteifungselement 40 mit den Zinken oder Vorsprüngen nicht radial nach außen von der Schaftwand 2 weg verlagert wird, sondern eine Horizontalverschiebung entlang der äußeren Oberfläche der Schaftwand 2 erfolgt. Die Vorsprünge oder blockartigen Absätze an der Schaftwand 2 werden durch die Verlagerung z.B. eines Verstellelementes durch eine Verstelleinrichtung in Kontakt oder außer Kontakt mit den Vorsprüngen oder Absätzen des Versteifungselementes 40 gebracht. Je nach Überdeckung und Ausfüllgrad der Zwischenräume zwischen den Blöcken an der Schaftwand 2 durch das Versteifungselement 40 vergrößert oder verringert sich die Steifigkeit und Formstabilität der Schaftwand 2 in diesem Bereich.

In der Figur 14 ist eine weitere Variante gezeigt, bei der an der Schaftwand 2 Noppen mit einem schwalbenschwanzförmigen Querschnitt angeordnet oder ausgebildet sind. Korrespondierend dazu sind an dem Versteifungselement 40 Vorsprünge in schwalbenschwanzförmiger Formgebung angeordnet. Somit weisen beiden Vorsprünge bzw. noppenartigen Elemente an dem Versteifungselement 40 und der Schaftwand 2 Hinterschneidungen auf, wodurch in dem dargestellten Zustand, in dem die Komponenten gezeigt sind, eine verbesserte Kraftübertragung stattfinden kann. Durch die mehrfache Keilform der Noppen bzw. der Formschlusselemente ist es möglich, die Komponenten leichter zu aktivieren und das Einführen und Ineingriffbringen miteinander zu erleichtern. Durch die keilförmige Formgebung ist zudem ein bewegungsabhängiger Steifigkeitssprung verwirklichbar. In der oberen Darstellung ist eine Darstellung eines Schnittes in Radialrichtung gezeigt, in der unteren Darstellung ist der Schnitt entlang der Linie A-A in der oberen Darstellung gezeigt, was einem Schnitt in Umfangsrichtung entspricht.

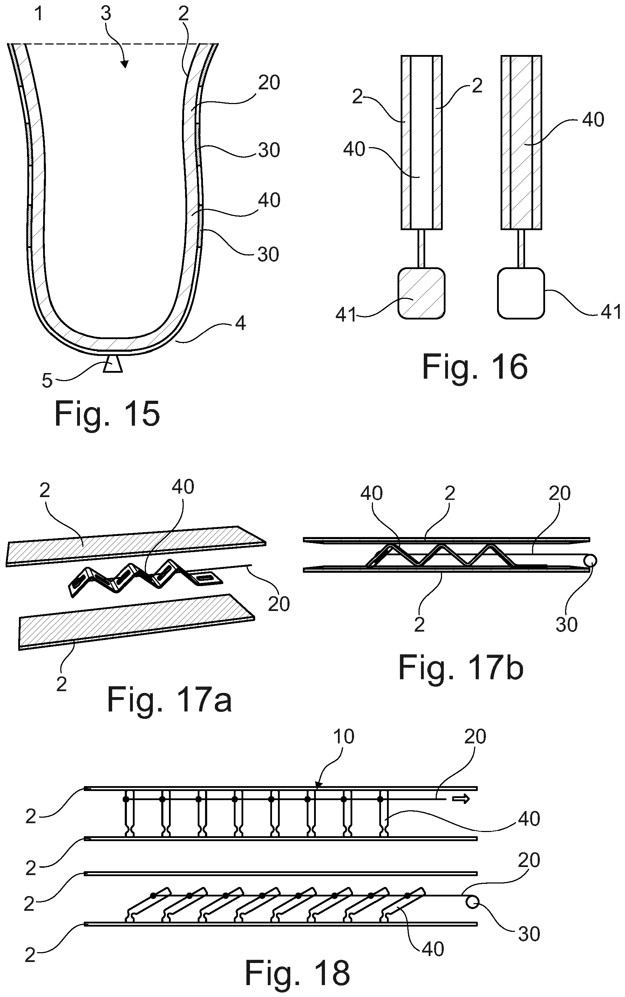

In der Figur 15 ist eine weitere Variante gezeigt, bei der der Schaft 1 als Prothesenschaft ausgebildet ist. Die Schaftwand 2 ist hohl ausgebildet und weist in ihrem Inneren eine magnetorheologische Flüssigkeit 40 als Versteifungselement auf. An der Außenseite des Prothesenschaftes 1 sind Elektromagnete als Verstelleinrichtungen 30 angeordnet, die auf das Fluidvolumen einwirken. Wird der Elektromagnet 30 aktiviert, verändert sich die Viskosität der magnetorheologischen Flüssigkeit 40, dadurch wird die Steifigkeit in dem jeweiligen Bereich verändert, insbesondere vergrößert. Die Volumina innerhalb der Schaftwand 2 können voneinander getrennt sein, sodass unterschiedliche magnetorheologische Flüssigkeiten in voneinander getrennten Volumina verwendet werden können, sodass konstruktiv unterschiedliche Viskositätsveränderungen und damit auch Steifigkeitsveränderungen bei gleichen Magnetfeldern anliegen. Die Magnete 30 können auch einzeln angesteuert werden, um Magnetfelder mit unterschiedlicher Feldstärke bereitzustellen, wodurch situationsbedingt und ortsabhängig unterschiedliche Steifigkeiten in dem Prothesenschaft 1 bereitgestellt werden.

In der Figur 16 ist eine Variante dargestellt, bei der das Versteifungselement 40 als ein Hohlraum oder Kissen ausgebildet ist, das als Zwischenlage zwischen zwei Schaftwänden 2 oder Schaftwandabschnitten angeordnet ist. Dem Kissen 40 ist ein Reservoir 41 zugeordnet, in dem ein Fluid, beispielsweise eine Flüssigkeit, angeordnet ist. Über eine Pumpe oder durch eine Verringerung des Volumens des Reservoirs, beispielsweise durch Zusammendrücken per Hand oder Muskelaktivierung, wird das Fluid aus dem Reservoir 41 in das Kissen 40 hineinbewegt und dort gehalten. Die Schaftwände 2 können elastisch ausgebildet sein, bei einem entleerten Kissen 40 ist die Steifigkeit vergleichsweise gering, bei einem gefüllten Kissen 40 wird sie vergrößert. Das Versteifungselement 40, gegebenenfalls mit einer Pumpe oder auch eingebettet zwischen zwei Blattfedern anstatt der Schaftwandbereiche, kann als komplettes Modul vorgefertigt und an oder in einer Schaftwand angeordnet und befestigt werden. Somit kann das Versteifungsmodul entweder als Zwischenlage zwischen zwei flexiblen Schaftwandbereichen oder an einer Außenseite der Schaftwand 2 befestigt werden.

Eine weitere Ausgestaltung ist in den Figuren 17a und 17b gezeigt. **In** der Figur 17a ist eine Explosionsdarstellung entweder eines Versteifungsmoduls oder einer Schaftwand mit zwei Schaftwandbereichen 2 gezeigt, zwischen denen ein Versteifungselement 40 in einer Wellenform oder einer zieharmonikaartigen Form angeordnet ist. Das Versteifungselement 40 ist beispielsweise aus einem federnden Blech ausgebildet. Dem Versteifungselement 40 ist ein Verstellelement 20 in Gestalt eines Zugmittels, beispielsweise eines Drahtes oder einer Schnur zugeordnet. In der Figur 17b ist schematisch die Verstelleinrichtung 30 in Gestalt eines Motors gezeigt, der eine Rolle antreibt, auf der das Verstellelement 20 aufgewickelt wird. Das rechte Ende des Versteifungselementes 40 ist ortsfest an einem Schaftwandbereich gelagert, das linke Ende kann nach rechts über das Verstellelement 20 verlagert werden. Wird über die Verstelleinrichtung 30 das Verstellelement 20 aufgewickelt, verlagert sich das linke Endes des Versteifungselementes in Richtung auf die Verstelleinrichtung 30, wodurch die im Wesentlichen geradlinigen Abschnitte zwischen den gebogenen oder gefalteten Bereichen des Versteifungselementes steiler ausgerichtet werden, wodurch sich eine vergrößerte Steifigkeit in diesem Bereich ergibt. Wird das Verstellelement 20 entspannt, kehrt aufgrund der elastischen Rückstellkräfte des Versteifungselementes 40 dieses in die Ausgangsposition zurück und verringert die Steifigkeit in diesem Bereich der Schaftwand 2.

Eine alternative Ausgestaltung ist in der Figur 18 gezeigt, bei der statt einer zieharmonikaartigen Ausgestaltung des Versteifungselementes 40 mehrere klappbare Versteifungselemente 40 zwischen den Wandabschnitten angeordnet sind. In der oberen Darstellung ist der Bereich 10 der Schaftwand versteift oder mit einer vergrößerten Steifigkeit versehen, während in der unteren Darstellung mit dem aufgewickelten Verstellelement 20 und der Verstelleinrichtung 30 als motorischer Antrieb die Versteifungselemente 40 geneigt sind, wodurch sich eine leichtere Verlagerbarkeit der Schaftwandbereiche 2 aufeinander ergibt. Die Versteifungselemente 40 sind vorteilhafterweise elastisch gelagert, sodass nach Wegfall einer Verstellkraft durch das Verstellelement 20 diese wieder in die ursprüngliche Stellung zurückkehren. Das Verstellelement 20 kann auch als zug- und druckkräfteübertragendes Element ausgebildet sein, um eine Verschwenkung in beide Richtungen zu bewirken.

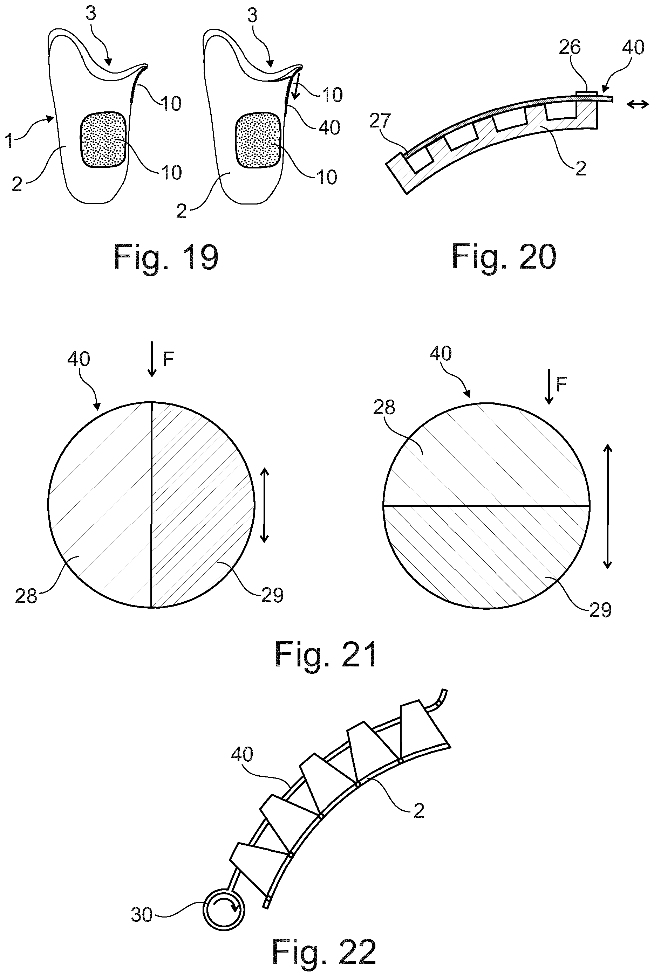

In der Figur 19 ist in einer Variante ein Prothesenschaft mit zwei hinsichtlich ihrer Steifigkeit veränderbaren Bereichen 10 gezeigt. Neben einem flächenartigen Funktionsbereich 10 in der Mitte des Prothesenschaftes 1 innerhalb der Schaftwand 2 ist an der sogenannten Ramusanlage am proximalen Rand im Bereich der Einstiegsöffnung 3 ein Versteifungselement 40 angeordnet, das über eine nicht dargestellte Verstelleinrichtung oder auch manuell verlagert werden kann. Das Versteifungselement 40 wird aus der in der linken Darstellung gezeigten Ausgangsstellung in die in der rechten Darstellung gezeigte Entlastungsstellung verlagert, sodass sich ein gesamter Bereich 10 am oberen Schaftrand hinsichtlich der Steifigkeit verändert. In dem oberen Bereich wird die Steifigkeit des Prothesenschaftes 1 verringert, beispielsweise um das Sitzen für einen Schaftnutzer angenehmer zu machen.

In der Figur 20 ist eine weitere Ausgestaltung gezeigt, bei der eine Versteifung der Schaftwand 2 über ein als Zugelement ausgebildetes Versteifungselement 40 erreicht wird. Wird das Versteifungselement 40, das an einem Befestigungspunkt 27 an der Schaftwand 2 festgelegt ist, an der Außenseite über Noppen oder Erhebungen von Nuten oder Rillen geführt gespannt, erhöht sich in diesem Zustand die Gesamtsteifigkeit der Schaftwand 2. Über eine Klemmeinrichtung 26 kann die einmal eingestellte Spannung innerhalb des Verstellelementes 20 fixiert werden. Wird das Zugelement oder das Versteifungselement 40 entspannt, wird die Vorspannung des Schaftwandbereiches, in dem das Zugelement gespannt wirksam ist, moduliert und verringert, wodurch die Gesamtsteifigkeit des Bereiches wieder verringert wird.

In der Figur 21 sind zwei unterschiedliche Orientierungen eines Versteifungselementes 40 gezeigt, das aus zwei Teilen 28, 29 besteht, die jeweils unterschiedliche Festigkeiten bzw. Steifigkeiten aufweisen. Die linke Hälfte 28 besteht aus einem weichen, nachgiebigen Material, während die rechte Hälfte 29 in der linken Darstellung aus einem fest, weniger nachgiebigen Material besteht. Wird eine Kraft F beispielsweise von oben auf das Versteifungselement 40 ausgeübt, ergeben sich je nach Stellung und Orientierung der beiden Hälften zueinander unterschiedliche Nachgiebigkeiten und Steifigkeiten. In der linken Darstellung wirken die beiden Hälften wie parallel zueinander angeordnete Federelemente. Aufgrund der Orientierung der Schnittebene in der Mitte des Versteifungselementes 40 parallel zur Kraftrichtung ergibt sich eine hohe Steifigkeit oder Festigkeit und ein hoher Widerstand gegen eine Verlagerung, was durch den kurzen Doppelpfeil angedeutet ist. Bei einer Drehung um 90° zur Orientierung der Kraftrichtung F, wie sie in der rechten Darstellung gezeigt ist, ergibt sich ein geringerer Biegewiderstand und somit eine geringere Festigkeit desjenigen Bereiches, in dem das Versteifungselement 40 angeordnet ist. Die Verdrehung zu der jeweiligen Kraftrichtung kann manuell oder motorisch oder durch einen anderen Antrieb erfolgen.

In der Figur 22 ist eine Variante der Figur 20 gezeigt, bei der ebenfalls ein Zugmittel als Versteifungselement 40 an einer Verstelleinrichtung 30, beispielsweise einer Rolle angeordnet ist. Die Verstelleinrichtung 30 kann manuell oder motorisch angetrieben sein und auf Grundlage eines Schalterbefehls oder von Sensordaten in die eine oder andere Richtung verlagert werden. Das der Verstelleinrichtung 30 abgewandte Ende des Versteifungselementes 40 ist an einem Vorsprung an der Schaftwand 2 gelagert und bildet ein Widerlager, sodass durch Aufwickeln oder Abwickeln des Zugelementes eine vergrößerte oder verringerte Spannung und Vorspannung in diesem Bereich erreicht wird.

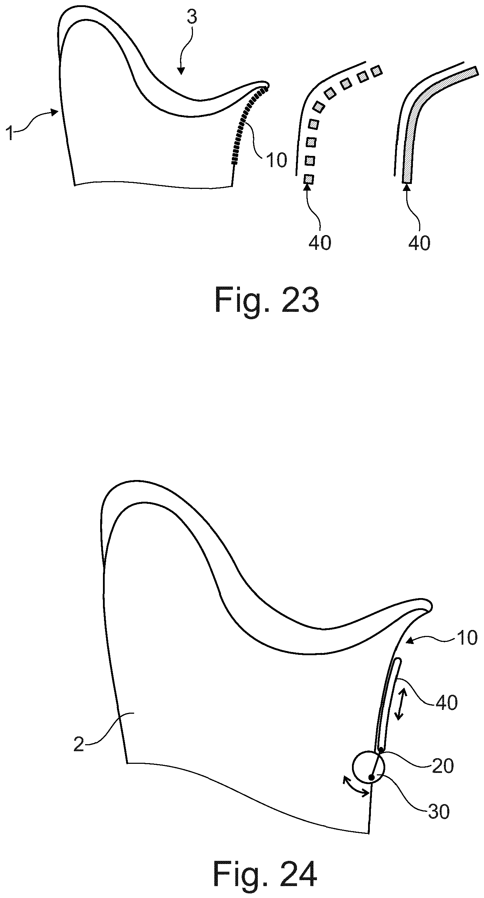

In der Figur 23 ist eine Variante mit einem Versteifungselement 40 in dem Bereich 10 des Schaftes 1 schematisch dargestellt. Der Prothesenschaft 1 ist in diesem Bereich grundsätzlich elastisch ausgebildet und weist eine Einlagerung oder ein Versteifungselement 40 mit magnetorheologischen Eigenschaften auf. In der rechten Darstellung sind zwei Zustände des Versteifungselementes 40 dargestellt, die linke Darstellung zeigt einen nicht polarisierten Zustand, in dem das Versteifungselement 40 sehr nachgiebig ist. In der rechten Darstellung ist das magnetorheologische Fluid polarisiert und wird aufgrund der erhöhten Viskosität einer Belastung einen höheren Widerstand entgegenstellen. Damit ist es möglich, dass in diesem Bereich Kräften ein größerer Verformungswiderstand entgegentritt. Bei Wegfall der Belastung und bei Wegfall der Polarisierung kehrt die Schaftwand 2 des Prothesenschaftes 1 wieder in die Ausgangsposition zurück, die von dem Orthopädietechniker ursprünglich eingestellt worden ist.

In der Figur 24 ist ein Versteifungsmodul an der Schaftwand 2 mit einer motorisch angetriebenen Verstelleinrichtung 30 gezeigt, an der ein Verstellelement 20 über einen Kurbelbetrieb gelagert ist. Je nach Drehrichtung ist es möglich, das Verstellelement 20 in Richtung auf den proximalen Schaftrand oder entgegengesetzt in distaler Richtung zu verlagern. An dem Verstellelement 20 ist ein Versteifungselement 40 angeordnet, das beispielsweise in einer Führung verschieblich an der Schaftwand 2 gelagert ist. Der Bereich 10 des Schaftes ist grundsätzlich nachgiebig ausgebildet und kann durch Verschieben des Versteifungselementes 20 nach oben versteift werden, sodass eine erhöhte Biegesteifigkeit bereitgestellt wird.

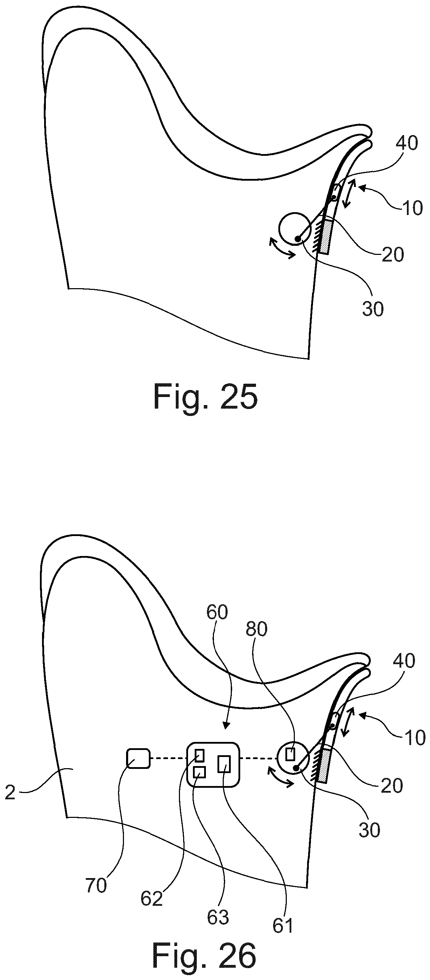

In der Figur 25 ist eine Variante der Figur 24 gezeigt, bei der das Versteifungsmodul auswechselbar an der Schaftwand 2 befestigt ist. Alternativ kann das Versteifungsmodul auch ohne Verstelleinrichtung 30 mit dem verschieblichen Verstellelement 20 und dem verschieblichen Versteifungselement 40 fest an der Außenseite oder innerhalb der Schaftwand 2 angeordnet sein. Durch Drehen der Verstelleinrichtung 30 wird über den Kurbeltrieb das Verstellelement 20 und das Versteifungselement 40 verlagert und die Steifigkeit des fest angeordneten Versteifungsmodules in diesem Bereich verändert.

In der Figur 26 ist eine weitere Ausführungsform dargestellt, die im Wesentlichen der der Figur 25 entspricht. Zusätzlich zu der Verstelleinrichtung 30 mit dem Versteifungselement 40, das verschiebbar entlang der Außenwand des Prothesenschaftes gelagert ist, ist in der Figur 26 eine Steuerungseinrichtung 60 dargestellt, die mit zumindest einem Sensor 70 gekoppelt ist. Die Kopplung kann drahtlos erfolgen. Alternativ oder ergänzend ist eine Kopplung des Sensors 70 oder der Sensoren 70 über ein Kabel oder über eine andere leitende Verbindung möglich und vorgesehen. Innerhalb der Steuerungseinrichtung 60 sind die notwendigen Komponenten zur Verarbeitung der Sensorsignale untergebracht, insbesondere eine Speicherkomponente 61, eine Datenverarbeitungseinrichtung 62 sowie eine Kommunikationsschnittstelle 63. Die Kommunikationsschnittstelle 63 kann Daten an externe Einrichtungen senden oder von dieser empfangen und zur Verarbeitung der Datenverarbeitungseinrichtung 62, beispielsweise einem Prozessor, zuleiten. Darüber hinaus sind auch die anderen, notwendigen Komponenten in der Steuerungseinrichtung 60 integriert oder damit gekoppelt, beispielsweise ein Energiespeicher, gegebenenfalls Verstärker oder andere elektrische, elektronische oder elektromechanische Komponenten.

Von der Steuerungseinrichtung 60 werden die notwendigen Aktivierungssignale und/oder Deaktivierungssignale der Verstelleinrichtung 30 übermittelt, die mit einem Aktuator 80 gekoppelt ist, der in dem dargestellten Ausführungsbeispiel als ein Elektromotor ausgebildet ist. Die Übermittlung kann auch über die Kommunikationsschnittstelle 63 erfolgen. Der Aktuator 80 verlagert beispielsweise eine Drehscheibe der Verstelleinrichtung 30 in die eine oder andere Richtung, sodass das Verstellelement 20 in Gestalt einer Schubstange das Versteifungselement 40 in die eine oder andere Richtung verlagert, um eine Veränderung der Steifigkeit eines Bereiches 10 der Schaftwand 2 zu bewirken.

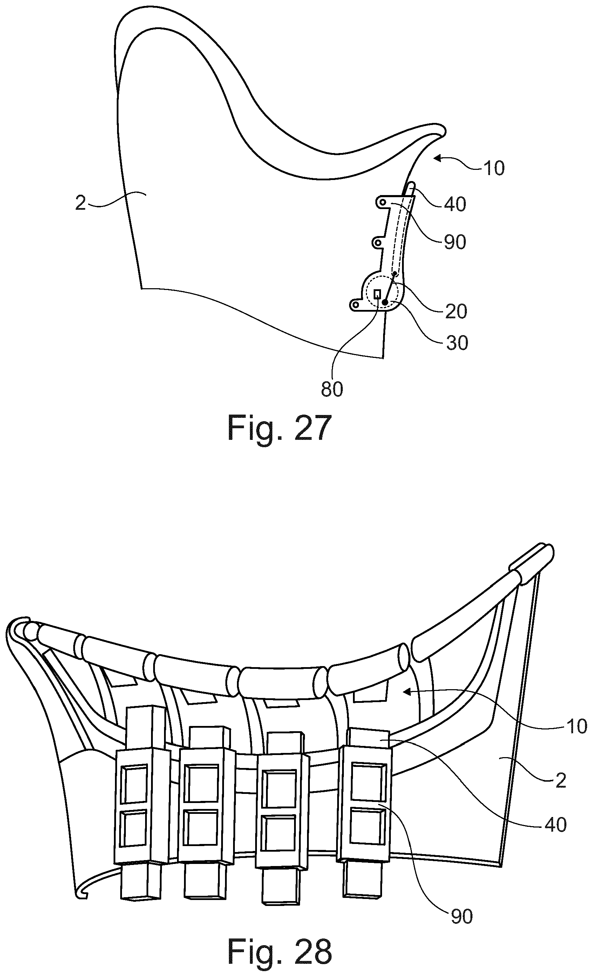

In der Figur 27 ist eine weitere Variante dargestellt, bei der sämtliche Komponenten zur Veränderung der Steifigkeit der Schaftwand 2 in einem Versteifungsmodul zusammengefasst sind. Das Versteifungsmodul weist ein Gehäuse auf, das über Schrauben oder Nieten oder andere Befestigungselemente an der Außenseite der Schaftwand 2 reversibel festlegbar ist. Das Versteifungsmodul kann auch in der Schaftwand 2 eingebettet werden oder in der Schaftwand 2 integriert sein. Das Gehäuse des Versteifungsmoduls dient gleichzeitig als Führung für das Versteifungselement 40, das in dem dargestellten Ausführungsbeispiel als eine längsverschiebliche Versteifungskomponente, beispielsweise als ein Schieber, eine verformbarer Leiste oder eine Feder, ausgebildet ist. Je nachdem, wie der Aktuator 80 die Verstelleinrichtung 30 mit dem Verstellelement 20 verlagert, wird das Versteifungselement 40 nach oben geschoben oder nach unten gezogen und verändert dadurch die Steifigkeit in dem proximalen Schaftwandbereich 10.

In der Figur 28 sind insgesamt vier Versteifungselemente 40 in Führungen 90 an der Außenseite der Schaftwand 20 angeordnet. Die Versteifungselemente 40 können nach oben oder unten verschoben werden, entweder manuell oder über einen nicht dargestellten Aktuator. Je weiter die Versteifungselemente 40 nach oben verlagert werden, desto steifer ist der proximalen Schaftwandbereich 10, der auch nachträglich in die Schaftwand 2 eingesetzt und daran befestigt werden kann. Dazu ist in der Schaftwand 2 eine entsprechende Ausnehmung eingearbeitet oder wird darin eingebracht, anschließend wird der Schaftwandbereich 10 insbesondere dauerhaft an der Schaftwand 2 befestigt, beispielsweise über Formschlusselemente, Befestigungselemente oder auch stoffschlüssig über Verschweißen, Verkleben oder ähnliches. Der Schaftwandbereich 10 weist jeweils Schlitze zwischen den einzelnen Versteifungselemente 40 auf, sodass die jeweiligen Bereiche separat voneinander hinsichtlich ihrer Steifigkeit verändert werden können.

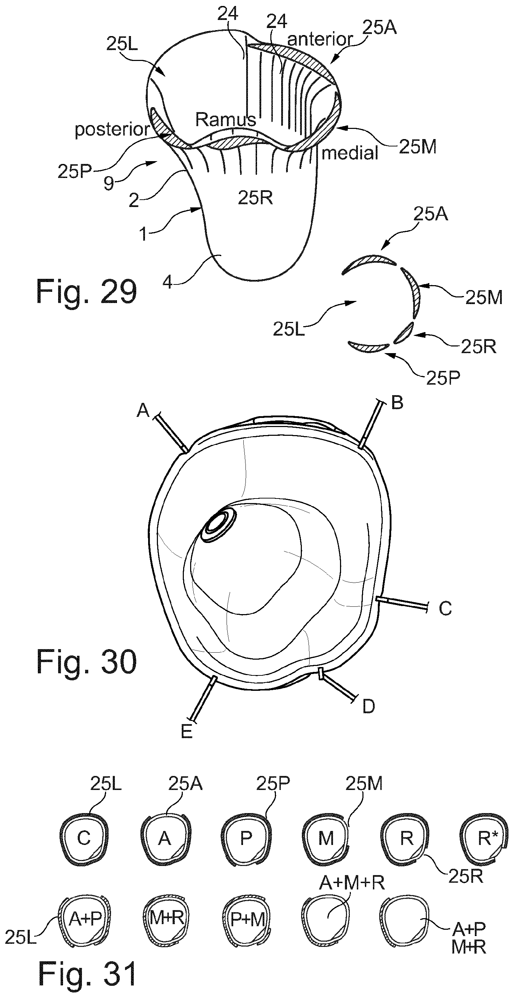

In der Figur 29 ist eine weitere Ausführungsform des Prothesenschaftes 1 dargestellt, der eine umlaufende Schaftwand 2 sowie einen distalen Endbereich 4 aufweist. Der distale Endbereich 4 und die durchgängige Schaftwand 2 bilden einen wesentlichen starren, hülsenförmigen Prothesenschaft 1 aus, in dessen proximalem Randbereich 9 von dem proximalen Schaftrand in distale Richtung verlaufende Ausnehmungen 24 bereichsweise eingebracht sind. Der Prothesenschaft 1 ist in insgesamt fünf Sektoren 25 unterteilt, wobei ein erster, lateraler Sektor 25 L keine Ausnehmungen 24 aufweist. Die anderen vier Sektoren sind ein anteriorer Sektor 25 A, ein medialer Sektor 25 M, ein posteriorer Sektor 25 P sowie ein Sektor 25 R für die Ramusanlage. In dem dargestellten Ausführungsbeispiel weisen bis auf den lateralen Sektor 25 L alle anderen Sektoren 25 A, 25 M, 25 R und 25 P Ausnehmungen 24 auf. Die Ausnehmungen 24 können sektorweise unterschiedlich breit, in einer unterschiedlichen Dichte und/oder in unterschiedlichen Längen ausgebildet sein. Je länger die Ausnehmungen 24 sich in distale Richtung erstrecken, desto nachgiebiger ist der proximale Randbereich 9 in diesem Sektor. Gleiches gilt für eine größere Breite der Ausnehmungen 24 sowie eine erhöhte oder hohe Anzahl an Ausnehmungen 24 pro Umfangslänge. Da der laterale Sektor 25 L hauptsächlich für die seitliche Führung und Stabilität zuständig ist und eine einstellbare Nachgiebigkeit oder Elastizität aufgrund der üblichen Bewegungen mit einem Prothesenschaft dort nicht notwendig ist, sind in den meisten Fällen keine Ausnehmungen in dem lateralen Sektor 25 L vorhanden. Alternativ oder ergänzend zu den Ausnehmungen 24 können unterschiedliche Materialstärken in den einzelnen Sektoren vorhanden sein, um die Nachgiebigkeit oder Elastizität der Schafteintrittsebene oder in dem proximalen Randbereich 9 einstellen zu können. In der rechten unteren Darstellung der Figur 29 ist eine Draufsicht auf den Prothesenschaft mit den einzelnen Sektoren 25 dargestellt. Die Ausrichtung und Benennung ergibt sich aus der Gehrichtung. In dem dargestellten Ausführungsbeispiel ist ein Prothesenschaft 1 für einen linken Stumpf dargestellt.

Die einzelnen Sektoren 25 A, 25 M, 25 R, 25 P und gegebenenfalls 25 L sind individuell in ihrer Steifigkeit veränderbar ausgebildet. Dabei ist es besonders vorteilhaft, nur den proximalen Schaftwandbereich 9 nachgiebig und schaltbar hinsichtlich der Steifigkeit auszugestalten, um in dem übrigen Bereich eine ausreichende Rigidität und Stabilität aufzuweisen. Die Einteilung und Dimensionierung der jeweiligen Sektoren kann individuell erfolgen. Ein Beispiel für eine Einteilung der Sektoren ist in der Figur 30 dargestellt, in der in Draufsicht ein Prothesenschaft gezeigt ist. Der erste Bezugspunkt A ist der Schnittpunkt für das Diagonalmaß und das laterale anterior-posterior Lager und bildet das anteriore Ende des lateralen Sektors 25 L. Das Diagonalmaß ist das Maß von der Ramusumgreifung zu dem Trochanter Major. Der Bezugspunkt B ist der Schnittpunkt der Vorderkante des Perineums zu dem medialen anterior-posterior Widerlager, der anteriorer Sektor 25 A liegt zwischen dem Bezugspunkt A und dem Bezugspunkt B. Der Bezugspunkt C liegt am Ramusaustritt am Übergang zum Perineum vor der Adduktoren-Abstützung, zwischen den Bezugspunkten B und C liegt der mediale Sektor 25 M. Der Sektor 25 R für die Ramusanlage liegt zwischen dem Bezugspunkt C und dem Bezugspunkt D, der die Hinterkante der Ramusumgreifung bildet. Der posteriorer Sektor 25 P befindet sich zwischen dem Bezugspunkt D und dem Bezugspunkt E, der gespiegelt zu dem Bezugspunkt die an der Laufrichtung festgelegt ist. Zwischen den Bezugspunkten A und D liegt der laterale Sektor 25 L.

In der Figur 31 sind 11 verschiedene Möglichkeiten zur Schaltung der nicht dargestellten Versteifungselemente über eine Verstelleinrichtung oder die Verstelleinrichtungen dargestellt. Oben links beginnend im Uhrzeigersinn ist eine vollständige Versteifung oder eine maximale Versteifung der einzelnen Sektoren vorhanden, sodass sich ein vollständig oder maximal versteifter Prothesenschaft in dem proximalen Randbereich ergibt. Bei einer Verringerung der Steifigkeit in dem anterioren Sektor 25 A ist die Rigidität in diesem Bereich verringert. Um den Stumpf weiterhin auch in diesem Bereich zu führen und bei einer Umschaltung von einer erhöhten Nachgiebigkeit auf eine erhöhte Steifigkeit zu verhindern, dass ein Teil oder ein Bereich des Stumpfes eingeklemmt wird, sind die zu versteifenden Sektoren oder der zu versteifende proximale Randbereich mit einem Innenschaft 26 versehen, der nachgiebig ausgebildet ist. Der Innenschaft 26 kann becherförmig mit einem distalen geschlossenen Bereich ausgebildet sein oder nur in dem Bereich des proximalen Randbereiches an der Schaftwand angeordnet sein. Eine erhöhte Nachgiebigkeit in dem hinteren Bereich wird durch eine Verringerung der Steifigkeit in dem posteriorer Sektor 25 P erzeugt, bei der Veränderung der Nachgiebigkeit in dem medialen Sektor 25 M werden die entsprechenden Versteifungselemente durch die Verstelleinrichtung in diesem Sektor entsprechend aktiviert oder deaktiviert, korrespondierend dazu erfolgt eine vollständige Freigabe oder eine maximale Verringerung der Steifigkeit durch Veränderung oder selektives Schalten der Steifigkeit in der Ramusanlage in dem Sektor 25 R. Hierzu gibt es zwei Möglichkeiten, bei der letzten Darstellung in der oberen Reihe der Figur 31 ist die Steifigkeit in dem Sektor der Ramusanlage 25 R minimiert und der Innenschaft oder die in Auskleidung nicht vorhanden oder besonders nachgiebig, sodass eine nahezu vollständig freie Beweglichkeit erreichbar ist, in der vorletzten Darstellung der oberen Reihe ist die Steifigkeit in dem Sektor 25 R reduziert, der Innenschaft ist jedoch noch vorhanden.

Sind mehrere Sektoren nachgiebig geschaltet, beispielsweise der anteriore Sektor 25 A, der mediale Sektor 25 M, der Sektor 25 R für die Ramusanlage und der posteriorer Sektor 25 P, ist eine maximale Nachgiebigkeit des Prothesenschaftes 1 vorhanden, zumindest in dem proximalen Randbereich 9, da nur noch der laterale Sektor 25 L in dem proximalen Randbereich 9 die ursprüngliche, maximale Steifigkeit aufweist. Ebenso ist es möglich, eine Verringerung der Steifigkeit in drei Sektoren, beispielsweise dem anterioren Sektor 25 A, dem medialen Sektor 25 M und dem Sektor 25 R für die Ramusanlage durchzuführen.

Alternativ können nur zwei Sektoren hinsichtlich der Steifigkeit selektiv verändert oder verringert werden, beispielsweise der mediale Sektor 25 M in Verbindung mit dem posterioren Sektor 25 P, der mediale Sektor 25 M mit dem Sektor 25 R für die Ramusanlage oder für eine seitliche Einfassung der anteriore Sektor 25 A und der posteriore Sektor 25 P.

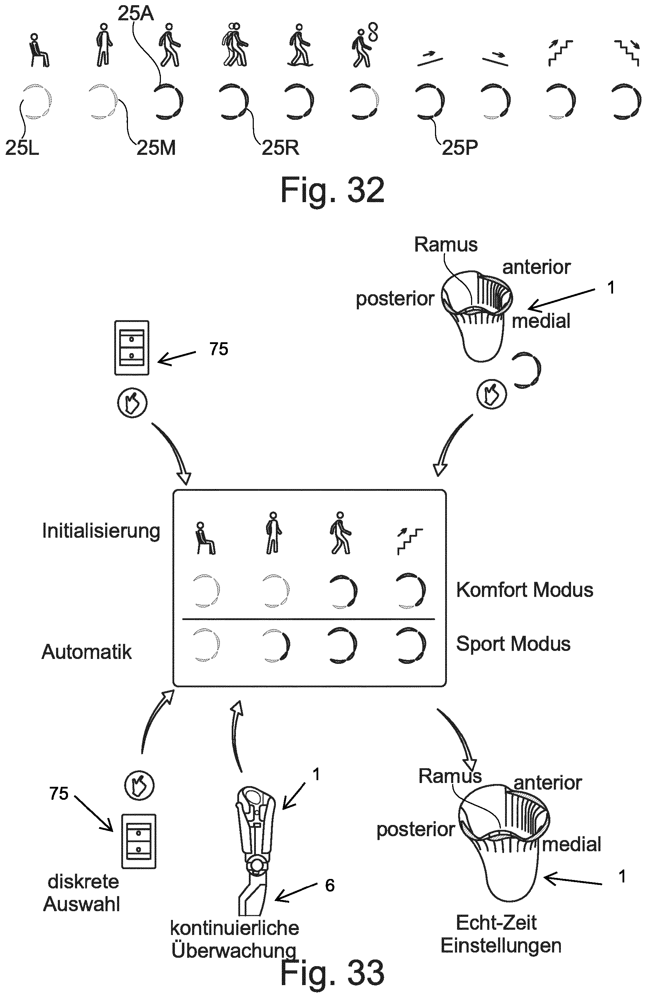

In der Figur 32 sind beispielhaft einige Situationen dargestellt, in denen die jeweiligen Sektoren hinsichtlich der Steifigkeit entsprechend eingestellt werden können. Die Einstellung kann für jede Person unterschiedlich sein, neben einer rein binären Umschaltung zwischen "steif" und "nachgiebig" können unterschiedliche Steifigkeiten innerhalb der Sektoren für unterschiedliche Gangsituationen oder Nutzungssituationen vorgesehen werden. Beim Sitzen und beim Stehen ist in dem dargestellten Ausführungsbeispiel vorgesehen, dass bis auf den lateralen Sektor, der in dem Ausführungsbeispiel nicht einstellbar ist, alle anderen Sektoren mit einer verringerten Steifigkeit eingestellt sind, beim Gehen oder schnellen Gehen ist in den vier anderen Sektoren eine erhöhte oder maximale Steifigkeit vorhanden, ebenso beim Gehen auf unebenem Gelände. Für das Gehen von Kurven ist der mediale Sektor 25 M nachgiebig geschaltet, beim Bergaufgehen sind wieder alle Sektoren versteift, beim Bergabgehen ebenso wie beim Treppe aufwärts Steigen ist der posteriore Sektor 25 P weich geschaltet, beim Treppe abwärts Gehen sind wiederum alle schaltbaren oder einstellbaren Sektoren mit einer verringerten Nachgiebigkeit oder einer erhöhten Steifigkeit versehen.

Die Einstellung der unterschiedlichen Modi oder Steifigkeiten in dem Bereich der Schafteintrittsebene in dem proximalen Randbereich 9 ist in der Figur 33 beispielhaft gezeigt. Für vier Situationen, beispielsweise das Sitzen, das Stehen, das Gehen sowie das Treppe aufwärts Gehen sind zwei unterschiedliche Modi vorgesehen, einmal den Komfortmodus, der in der oberen Zeile dargestellt ist, und der darunter dargestellte Sportmodus. Beide Modi können manuell über eine Eingabevorrichtung 75 verändert werden, sodass für jeden Patienten in jeder Situation die als angenehm empfundene Einstellung vorhanden ist. Eine Umschaltung zwischen einem Komfortmodus und dem Sportmodus kann automatisch oder durch manuelle Umschaltung mit der Eingabevorrichtung 75 erfolgen. Die Bewegungen des Nutzers werden kontinuierlich über die Sensoren überwacht, um automatisch Änderungen in dem Nutzungsverhalten oder die unterschiedlichen Bewegungssituationen zu erkennen. Die Überwachung erfolgt über Sensoren, die sowohl in dem Prothesenschaft 1 als auch in den anderen prothetischen Komponenten 6 oder auch nur in den anderen prothetischen Komponenten vorhanden sein können. Die Einstellung der Steifigkeit der Sektoren in dem proximalen Randbereich oder auch der Steifigkeit des gesamten proximalen Randbereiches erfolgt vorteilhafterweise in Echtzeit, um stets eine angepasste Steifigkeit des Prothesenschaftes 1 bereitstellen zu können.

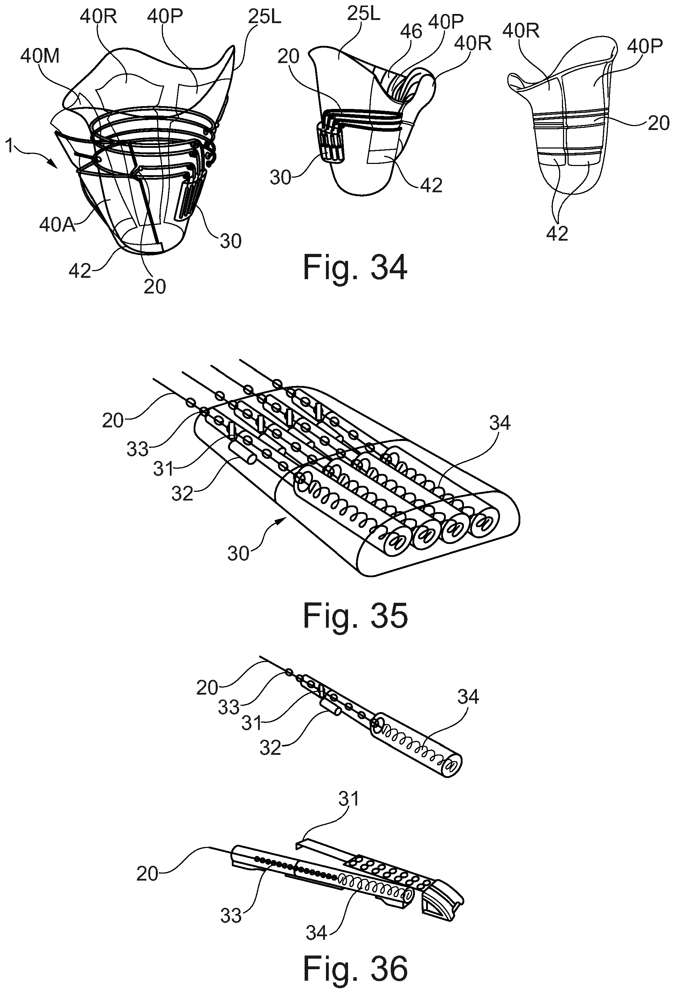

Ein Ausführungsbeispiel zur Einstellung von sektorweisen oder bereichsweisen Steifigkeiten in der Schafteintrittsebene oder in dem proximalen Randbereich ist in der Figur 34 dargestellt, in der drei Darstellungen eines Ausführungsbeispiels eines Prothesenschaftes 1 gezeigt sind. Der Prothesenschaft 1 weist einen, den nicht dargestellten Stumpf vollständig umgebenden, Innenschaft 26 auf, an dessen Außenseite oder in dem in Nuten oder Kanälen ein Seilzugsystem mit vier Seilzügen angeordnet ist. Die Seilzüge sind die Verstellelemente 20, die von einer zentralen Verstelleinrichtung 30 an der Außenseite der Schaftwand 2 betätigt werden. Jedes der vier Seile ist umlaufend oder zumindest teilweise umlaufend um den Prothesenschaft angeordnet und weist ein festgelegtes Ende und ein verstellbares Ende an der Verstelleinrichtung 30 auf. Jedes Seil ist so geführt, dass es genau ein Schaftwandsegment als Versteifungselement 40 aktiviert. Jedes Schaftwandsegment ist als Schale ausgebildet und an der Außenseite des Prothesenschaftes 1 angeordnet, korrespondierend zu jeweils einem Sektor. In dem dargestellten Ausführungsbeispiel sind vier Schaftwandsegmente 40 A, 40 M, 40 R 40 P, also anterior, medial, in dem Bereich der Ramusanlage und posteriorer positioniert. Die Verstelleinrichtung 30 ist an einem lateralen Bereich des Prothesenschaftes 1 befestigt. Der Prothesenschaft 1 ist ausreichend stabil, um eine Seilführung an ihm oder in ihm zu ermöglichen, die bei Spannen des Seils keine Veränderung des Innenumfanges oder nur eine vernachlässigbar geringe Verringerung oder Verformung des Innenraumes des Prothesenschaftes 1 zur Folge hat. Wird ein Seilzug 20 gespannt, wird das jeweilige Schaftwandsegment 40 A, 40 M, 40 R, 40 P, an dessen Außenseite der Seilzug 20 als Verstellelement entlang geführt ist, in Richtung auf den Stumpf gezogen und verhindert oder verringert eine Verformung des Prothesenschaftes 1 in diesem Bereich lateral nach außen. Bis zu dem jeweiligen Schaftwandsegment und danach verläuft das jeweilige Seil entkoppelt von dem Schaftwandsegment, z.B. verläuft der Seilzug innerhalb der Schaftwand oder an der Außenseite zwischen dem Prothesenschaft der Innenseite des Schaftwandsegmentes.

In der linken Darstellung der Figur 34 sind die Schaftwandsegmente 40 oder Schalen distal gelenkig mit dem Prothesenschaft 1 verbunden, beispielsweise über ein Textil oder ein Gelenk 42. Ist das jeweilige Schaftwandsegment 40 A, 40 M, 40 R, 40 P entriegelt, kann es sich in einem begrenzten Maß von der Schaftwand des Prothesenschaftes 1 abheben, sodass es möglich ist, dass der Prothesenschaft in dem proximalen Randbereich 9 flexibel, insbesondere elastisch nachgibt. In der mittleren Darstellung der Figur 34 ist dargestellt, dass der Prothesenschaft 1 an der Außenseite und distal zu dem lateralen Sektor 25 L mit der Verstelleinrichtung 30 versehen ist, von der die vier Seilzüge umlaufend um den Prothesenschaft herumgeführt sind, zumindest bis jeweils hinter das zu bewegende Schaftwandsegment. Es sind ein posteriores Schaftwandsegment 40 P und das Schaftwandsegment 40R für die Ramusanlage gezeigt, das Schaftwandsegment 40 R ist gelöst. Aufgrund des Gelenkes 42 an dem distalen Bereich des Schaftwandsegmentes, das als Textil oder ein anderes Festkörpergelenk ausgebildet ist, kann Schaftwandsegment 40 R von dem Rest des Prothesenschaftes radial nach außen verlagert werden, sodass sich der jeweilige Bereich des Prothesenschaftes mit dem Sektor 25 R und gegebenenfalls einem Innenschaft bei einer Belastung durch den Stumpf leichter nach außen verlagern lässt als mit einem angelegten Schaftwandsegment. Die rechte Darstellung gezeigt die beiden anderen Segmente, ein anteriores Schaftwandsegment 40 A und ein mediales Schaftwandsegment 40M, die beide an der Außenseite des Prothesenschaftes 1 mit den Segmenten und Ausnehmungen 24 anliegen.

Die Schaftwandsegmente 40A, 40 M, 40P und 40R bilden einen Teil einer Außenschale des Prothesenschaftes und werden über die zentrale Verstelleinrichtung 30, die in der Figur 35 vergrößert dargestellt sind, über die Seilzüge aktuiert. Die Seilzüge als Verstellelemente 20 sind in dem dargestellten Ausführungsbeispiel über Federn 34 vorgespannt und können über einen Aktuator 32 entriegelt oder verriegelt werden. Aufgrund der Federn 34 werden die Seilzüge 20 auf Spannung an dem Schaft gehalten. Wird ein Seilzug 20 entriegelt, kann das Schaftwandsegment, das mit dem Seilzug gekoppelt ist, nach außen verschenkt werden. In der nach außen verschwenkten Stellung, in der die Feder 34 gespannt ist, kann der Seilzug verriegelt werden, sodass eine dauerhafte Lockerung und damit eine Verringerung der Steifigkeit in dem Sektor, dem das jeweilige Segment zugeordnet ist, erreicht werden. Wird die Verriegelung aufgehoben, zieht sich die Feder 34 wieder zusammen und bewegt das Schaftwandsegment in Richtung auf den Stumpf. In der maximal verkürzten Stellung der Federn 34 ist die effektive Länge des Seilzuges maximal verkürzt, so dass das damit verbundene Schaftwandsegment maximal angepresst ist. Wird in dieser Stellung der Seilzug verriegelt, ist in dem entsprechenden Segment die maximale Steifigkeit in dem proximalen Endbereich eingestellt.

In der Figur 36 sind zwei Ausführungsbeispiele für die Freigabe bzw. das Verriegeln des jeweiligen Seilzuges bzw. der Federverspannung gezeigt. Bei der oberen Darstellung wirkt eine Zugfeder 34 auf das Seil 20, an dem Kugeln 33 oder Vorsprünge befestigt sind, die mit einem Verriegelungselement 31 in Eingriff treten. Das Verriegelungselement 31 ist als schwenkbarer Hebel ausgebildet, der über einen Aktuator 32, beispielsweise ein Magnetschalter oder dergleichen, aus einer Freigabestellung, die dargestellt ist, in eine Verriegelungsteil, die durch den Pfeil angedeutet ist, und zurück bewegbar ist. Eine alternative Variante ist in der unteren Darstellung der Figur 36 gezeigt, bei der ein Verriegelungselement 31 aus der dargestellten Freigabestellung nach unten in eine Verriegelungsstellung bewegt wird, um die Kugeln 33 oder Vorsprünge an einer Bewegung in Richtung der Längserstreckung des Seilzuges 20 oder entlang der Kraftwirkung der Feder 34 zu sperren.

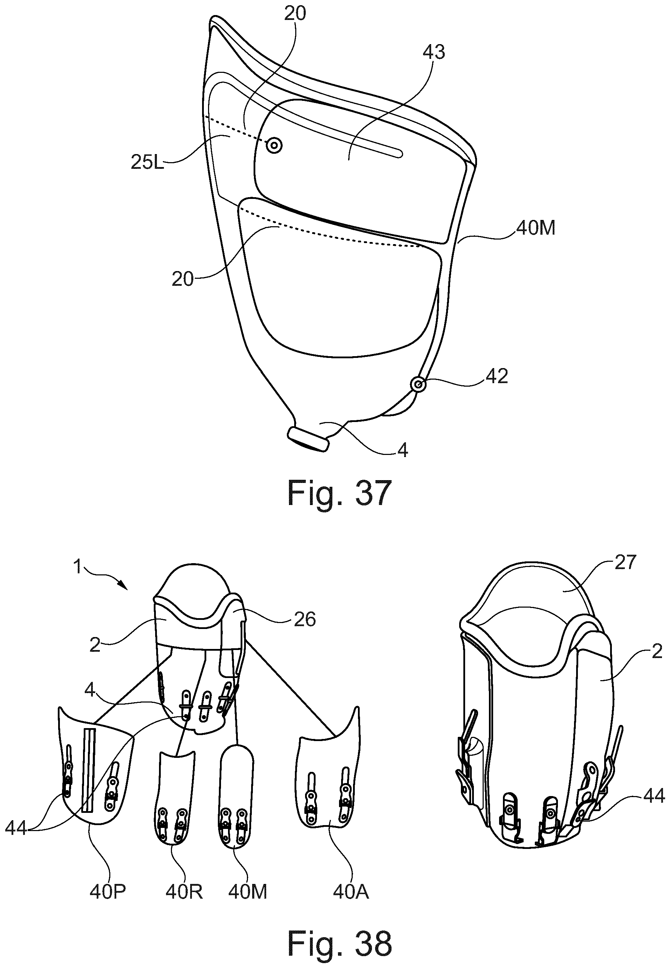

In der Figur 37 ist eine weitere Variante des Prothesenschaftes 1 dargestellt, bei dem flexible Bereiche 43 in dem proximalen Randbereich zusammen mit rigiden, schwenkbar an dem distalen, geschlossenen Prothesenschaft befestigten Schaftwandsegmenten 40 M eingesetzt sind, um unterschiedliche Steifigkeiten in dem proximalen Schaftwandbereich zu erreichen. Neben dem starren lateralen Sektor 25 L sind in einem Ausführungsbeispiel in dem anterioren Sektor und dem posterioren Sektor flexible Sektoren 43 vorgesehen, die über Seilzüge oder andere Verstellelemente 20 zwischen einem flexiblem Verhalten und einem elastischen Verhalten umschaltbar sind. Flexible Bereiche 43 in der Schafteintrittsebene oder in dem proximalen Randbereich bieten eine nach innen gerichtete Flexibilität. Die Verstellelemente 20 in Gestalt von Seilzügen sind innerhalb der flexiblen Bereich 43 angeordnet und können gespannt oder entspannt werden. In dem gespannten Zustand ist der entsprechende Sektor mit dem flexiblen Bereich 43 nach innen flexibel und nach außen nicht verlagerbar. In einem entspannten Zustand ist auch eine Nachgiebigkeit nach radial außen gegeben, um das Trageverhalten an die jeweiligen Bedürfnisse des Nutzers anzupassen. Solche flexiblen Bereiche 43 können mit den rigiden Schaftsegmenten, die anhand der Figur 34 erläutert worden sind, kombiniert werden.

In der Figur 38 ist ein Ausführungsbeispiel eines Prothesenschaftes 1 mit einem rigiden distalen Endbereich und vier Schaftwandsegmenten 40 dargestellt, die rigide ausgebildet sind und über Schnellverschlüsse 44 an der Schaftwand 2 an der Außenseite des Prothesenschaftes 1 montiert und demontiert werden können. An der Innenseite des Prothesenschaftes 1 ist ein Innenschaft 26 angeordnet, um eine glattwandigen Innenoberfläche für die Aufnahme eines Stumpfes bereitzustellen. Der laterale Sektor ist rigide und einstückig an dem distalen Endbereich 4 ausgebildet, die anderen Schaftwandsegmente 40 A, 40, 40 R, 40 P sind separat an dem Prothesenschaft anbringbar und verändern die Steifigkeit in dem jeweiligen Bereich.

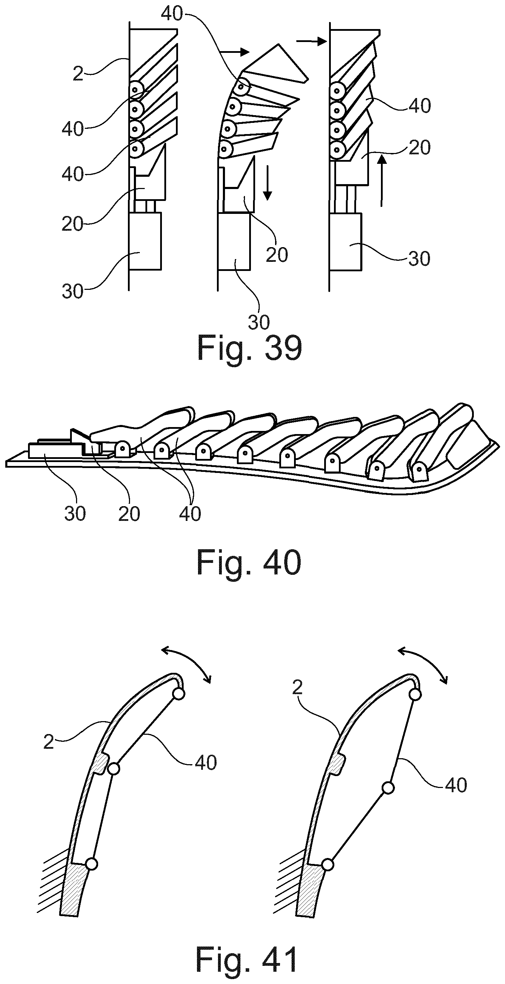

In der Figur 39 ist in einer schematischen Detaildarstellung eine weitere Ausführungsform des Prothesenschaftes mit einer Schaftwand 2 dargestellt, an der eine Verstelleinrichtung 30 als Aktuator mit einem Verstellelement 20 gekoppelt ist. Die Verstelleinrichtung 30 bewegt das Verstellelement 20 in Pfeilrichtung nach oben bzw. nach unten und wirkt auf in proximal-distal Richtung hintereinander angeordnete, schwenkbar gelagerte Versteifungselemente 40 ein. In der linken Darstellung liegen die Versteifungselemente 40 nicht aneinander an und erlauben eine Beweglichkeit nach rechts bzw. nach außen bis zu dem Zeitpunkt oder Zustand, an dem alle Versteifungselemente 40 aneinander anliegen. In der mittleren Darstellung ist das Verstellelement 20 nach unten verlagert, sodass der Abstand zwischen den Versteifungselementen und der mögliche Verlagerungsweg für die Schaftwand 2 vergrößert wird, sodass die Schaftwand 2 mit dem oberen, keilförmigen Anlageelement weiter nach außen verlagert werden kann. In der rechten Darstellung der Figur 39 befindet sich das Verstellelement 20 in der maximal proximalen Stellung, sodass alle Versteifungselemente 40 in dieser Position aneinander anliegen und ebenfalls an dem keilförmigen Anlageelement anliegen. Eine Bewegung nach rechts oder radial nach außen ist damit verhindert oder zumindest erschwert.

In der Figur 40 ist eine Variante dieser schuppenartigen Ausgestaltung dargestellt, bei der das dem Verstellelement 20 zugeordnete Versteifungselemente 40 als Doppelarmiger Hebel ausgebildet ist, der auf der einen Hebelseite auf dem nachfolgend angeordneten Versteifungselement 40 aufliegt und auf der anderen Seite von dem keilförmigen Verstellelement 20 in seiner Stellung verändert wird. Die maximale Versteifung wird erreicht, wenn das Verstellelement 20 durch die Verstelleinrichtung 30 maximal in Richtung auf das erste Verstellelement 40 verlagert worden ist. Alle nachfolgenden Versteifungselemente 40 liegen dann aufeinander an und drücken das proximale Anlageelement nach unten, sodass sich eine maximale Versteifung ergibt. Wird das Verstellelement 20 nach links verlagert, ist entsprechend mehr Spiel und Nachgiebigkeit in dem als Modul aufgebauten System.

In der Figur 41 ist eine weitere Ausführungsform des orthopädietechnischen Schaftes in einer Detaildarstellung gezeigt, bei der an der Schaftwand 2 ein Versteifungselement 40 angeordnet, dass als ein Kniehebel ausgebildet ist. In der linken Darstellung der Figur 41 liegt das 2-teilige Versteifungselement 40 an einem Anschlag an der Außenseite der Schaftwand 2 an und verhindert bei einer radial nach außen wirkenden Belastung, in dem dargestellten Ausführungsbeispiel nach rechts wirkenden Belastung, eine weitere Verlagerung des proximalen Schaftrandes oder behindert dies zumindest wesentlich. In der rechten Darstellung in der Figur 41 ist das Versteifungselement 40 über den Druckpunkt hinaus nach außen verkippt, wobei der mittlere Anlagepunkt an dem Anlageelement von diesem wegbewegt wurde, beispielsweise motorisch über eine nicht dargestellte Verstelleinrichtung. Wirkt dann eine radial nach außen gerichtete Kraft an der proximalen Schaftwand, verlagern sich die beiden Elemente des Kniehebels um das in der Mitte angeordnete Lager und ermöglichen eine Verformung des Prothesenschaftes in diesem Bereich oder erleichtern diese aufgrund der verringerten Steifigkeit.

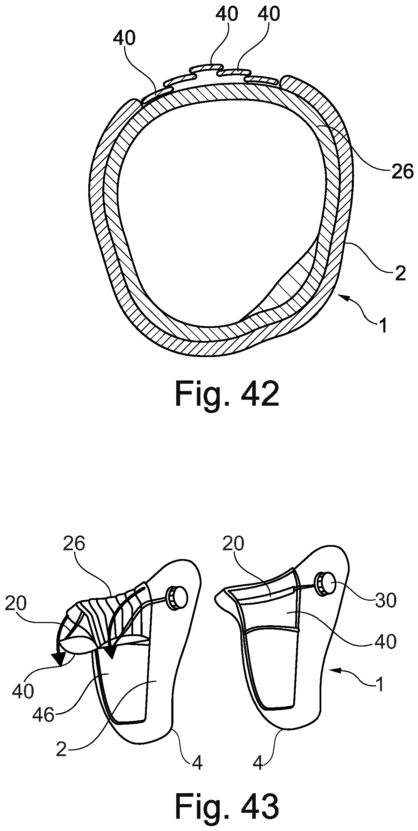

In der Figur 42 ist in einer Schnittansicht in Draufsicht der Prothesenschaft 1 mit der Prothese Schaftwand 2 dargestellt. Auf der Innenseite ist ein Innenschaft 26 angeordnet, der den Stumpf vollständig radial umgibt. Der grundsätzliche Aufbau des Prothesenschaftes 1 entspricht dem der Figur 34, grundsätzliche sind auch andere Ausführungsformen möglich. In einem Schaftwandsektor, in dem dargestellten Ausführungsbeispiel in dem anterioren Schaftwandsektor, sind Lamellen angeordnet, die einander überlappen. Durch die Überlappung der Lamellen, die die Versteifungselemente 40 ausbilden, ist es möglich, über den gesamten Bereich bzw. innerhalb des gesamten Schaftwandsektors die Steifigkeit durch Manipulation einer einzelnen Lamelle zu manipulieren. Wird beispielsweise die zentrale Lamellen steif geschaltet, beispielsweise mit einem Versteifungselement wie es in den Figuren 17 und 18 gezeigt ist, blockiert dieses Versteifungselement ein nach außen Biegen auch der benachbarten Lamellen. Alternativ oder ergänzend zu einer Versteifung der einzelnen Lamellen ist es möglich und vorgesehen, dass die Kontaktstelle der Lamellen sensorgesteuert und reversibel verändert werden kann. Beispielsweise wird die Haftreibung in den Überlappungsbereichen und Kontaktbereichen der einzelnen Lamellen reversibel geändert. Wird eine Relativbewegung der einzelnen Lamellen zueinander verhindert, beispielsweise durch unterschiedlich haftende Zwischenelemente, durch das temporäre Abdecken von haftenden Zwischenelementen oder durch elektromagnetische Verblockung der Lamellen in dem Überlappungsbereich der Lamellen, wird der gesamte Lamellenverbund aus den einander überlappenden Lamellen versteift und dient somit als Versteifungselement.

In der Figur 43 ist eine weitere Ausgestaltung des Prothesenschaftes 1 mit einer Schaftwand 2 dargestellt, bei der ebenfalls ein geschlossener distaler Endbereich 4 ausgebildet ist, an dem nicht näher dargestellte Befestigungseinrichtungen für orthopädietechnische, insbesondere prothetische Komponenten angeordnet oder ausgebildet sind. Innerhalb der Schaftwand 2 ist ein Fenster oder Ausschnitt 46 vorhanden, in dem dargestellten Ausführungsbeispiel in dem anterioren Bereich, der mit einem Material ausgefüllt ist oder an dem eine Abdeckung angebracht ist, um bis zu dem proximalen Endbereich eine in Umfangsrichtung geschlossene Schaftwand 2 bereitzustellen. An dem proximalen Ende des Ausschnittes 46 ist eine Klappe als Versteifungselement 40 schwenkbar angeordnet. Die Klappe ist in der linken Darstellung der Figur 43 geöffnet, sodass sich der Innenschaft 26, der in diesem Bereich lamellenartig ausgebildet ist, leicht nach außen verlagern lässt. Der Innenschaft 26 muss nicht lamellenartig ausgebildet sein. In der rechten Darstellung ist das Versteifungselement 40 über die Verstelleinrichtung 30 und das Verstellelement 20 in die geschlossene, in Richtung auf den nicht dargestellten Stumpf bewegte Stellung verlagert. In dieser Stellung ist der Prothesenschaft 1 auch in dem proximalen Randbereich im Wesentlichen starr, da das Verstellelement 20 als Seilzug oder Gurt flexibel und im Wesentlichen zugstarr ausgebildet ist. Die übrige Schaftwand 2 ist starr oder rigide. In einer Variante der Figur 43 ist die Schaftwand 2 ohne einen dahinterliegenden Innenschaft 26 ausgebildet, sodass die nach außen verlagerte Klappe als Versteifungselement 40 den Umfang des Prothesenschaftes 1 in dem proximalen Randbereich vergrößert und dadurch in diesem Sektor oder Bereich die Steifigkeit minimiert. Die Schaftwand 2 in diesem Bereich oberhalb des Ausschnittes 46 ist verlagerbar und vermindert dort die Steifigkeit auf ein Minimum. Der Ausschnitt 46 reicht dann bis zu dem oberen, proximalen Rand und bildet in dem proximalen Randbereich ein Fenster, das durch die Klappe als Versteifungselement 40 verschlossen bzw. geöffnet werden kann.

### Bezugszeichenliste

- 1 -: Schaft
- 2 -: Schaftwand
- 3 -: Einstiegsöffnung
- 4 -: Distaler Endbereich
- 5 -: Befestigungseinrichtung
- 6 -: Prothesenkniegelenk
- 7 -: Unterschenkelrohr
- 8 -: Prothesenfuß
- 9 -: Proximaler Randbereich
- 10 -: erster Bereich
- 11 -: Fußpunkt
- 12 -: Dämpferelement
- 20 -: Verstellelement
- 21 -: Innerer Schaftwandbereich
- 22 -: Äußerer Schaftwandbereich
- 23 -: Schaftwandsegment
- 24 -: Ausnehmung
- 25 -: Sektor
- 26 -: Innenschaft
- 27 -: Schaftwandsegment
- 28 -: Erster Teil
- 29 -: Zweiter Teil
- 30 -: Verstelleinrichtung
- 31 -: Verriegelungselement
- 32 -: Aktuator
- 33 -: Kugel
- 34 -: Feder
- 35 -: Schräge
- 40 -: Versteifungselement
- 41 -: Reservoir
- 42 -: Gelenk
- 43 -: Flexibler Bereich
- 44 -: Schnellverschlüsse
- 45 -: Schräge
- 46 -: Ausschnitt
- 60 -: Steuerungseinrichtung
- 61 -: Speicherkomponente
- 62 -: Datenverarbeitungseinrichtung
- 63 -: Kommunikationsschnittstelle
- 70 -: Sensor
- 80 -: Aktuator
- 90 -: Führung

## Patentansprüche

1. Orthopädietechnischer Schaft zur Anlage an und Aufnahme von einem Gliedmaßenstumpf oder einer Gliedmaße, mit
- zumindest einer Schaftwand (2),
- einer proximalen Einstiegsöffnung (3),
- einem distalen Ende (4) und
- zumindest einer Befestigungseinrichtung (5), die zur Festlegung zumindest einer distalen orthopädietechnischen Komponente (6) an dem Schaft (1) angeordnet ist, **dadurch gekennzeichnet, dass** an oder in der Schaftwand (2) zumindest ein Versteifungselement (40) angeordnet oder ausgebildet ist, dem eine Verstelleinrichtung (30) zugeordnet ist und über das eine Steifigkeit der Schaftwand (2) zumindest bereichsweise reversibel veränderbar ist.

2. Orthopädietechnischer Schaft nach Anspruch 1, **dadurch gekennzeichnet, dass** das Versteifungselement (40) verlagerbar in oder an der Schaftwand (2) gelagert ist.

3. Orthopädietechnischer Schaft nach Anspruch 1 oder 2, **dadurch gekennzeichnet, dass** die Verstelleinrichtung (30) an oder in der Schaftwand (2) angeordnet ist.

4. Orthopädietechnischer Schaft nach einem der voranstehenden Ansprüche, **dadurch gekennzeichnet, dass** das Versteifungselement (40) über ein Verstellelement (20) mit der Verstelleinrichtung (30) gekoppelt ist und das Verstellelement (20) als Zahnrad, Rolle, Kurbel, Hebel, Zugmittel, Drehelement, Kolben, Schubmittel oder Ventil ausgebildet ist.

5. Orthopädietechnischer Schaft nach einem der voranstehenden Ansprüche, **dadurch gekennzeichnet, dass** die Verstelleinrichtung (30) als Elektromagnet, Pumpe, Elektromotor, Heizung, Kühlelement, Piezoelement, elektroaktives Polymer, Kraftspeicher oder manuell betätigbare Einrichtung ausgebildet ist.

6. Orthopädietechnischer Schaft nach einem der voranstehenden Ansprüche, **dadurch gekennzeichnet, dass** das Versteifungselement (40) als Fluidvolumen, Formgedächniselement, Wellkörper, Zieharmonikaelement, Verzahnungskörper, Feder, Schaumkörper, Phasenwechselmaterial, Klappelement, Dämpfer oder Elektroadhäsionselement ausgebildet ist.

7. Orthopädietechnischer Schaft nach einem der voranstehenden Ansprüche, **dadurch gekennzeichnet, dass** die Verstelleinrichtung (30) einen Aktuator (80) aufweist, der über eine ihm zugeordnete Steuerungseinrichtung (60) aktivierbar und deaktivierbar ist, die mit zumindest einem Sensor (70) oder Schalter gekoppelt ist und auf der Grundlage von Sensordaten oder einer Schalterbetätigung den Aktuator aktiviert oder deaktiviert.

8. Orthopädietechnischer Schaft nach Anspruch 7, **dadurch gekennzeichnet, dass** der zumindest eine Sensor (70) als Temperatursensor, Beschleunigungssensor, IMU, Raumlagesensor, Neigungssensor, Kraftsensor, optischer Sensor, Ableiterelektrode, Drucksensor, DMS oder Widerstandssensor ausgebildet ist.

9. Orthopädietechnischer Schaft nach Anspruch 7 oder 8, **dadurch gekennzeichnet, dass** der Steuerungseinrichtung (60) ein Speicher (61), eine Datenverarbeitungseinrichtung (62) und/oder eine Kommunikationsschnittstelle (63) zugeordnet ist.

10. Orthopädietechnischer Schaft nach einem der voranstehenden Ansprüche, **dadurch gekennzeichnet, dass** in oder an der Schaftwand (2) eine Aufnahme (90) für die Verstelleinrichtung (30) und/oder das Versteifungselement (40) angeordnet ist.

11. Orthopädietechnischer Schaft nach einem der voranstehenden Ansprüche, **dadurch gekennzeichnet, dass** das Versteifungselement (40) und die Verstelleinrichtung (30) oder das Verstellelement (20), die Verstelleinrichtung (30) und das Versteifungselement (40) als ein Versteifungsmodul zusammengefasst sind.

12. Orthopädietechnischer Schaft nach einem der voranstehenden Ansprüche, **dadurch gekennzeichnet, dass** in einem proximalen Randbereich (9) der Schaftwand (2) Sektoren (25) mit unterschiedlicher radialer Elastizität ausgebildet sind.

13. Orthopädietechnischer Schaft nach Anspruch 12, **dadurch gekennzeichnet, dass** die Sektoren (25) unterschiedliche Materialien, unterschiedliche Materialstärken, unterschiedlich breite Ausnehmungen (24) und/oder eine unterschiedliche Dichte an Ausnehmungen (24) aufweisen.

14. Orthopädietechnischer Schaft nach Anspruch 12, **dadurch gekennzeichnet, dass** den einzelnen Sektoren (25) verlagerbare Schaftwandsegmente (40A, 40 M, 40R, 40P) als Versteifungselemente (40) zugeordnet sind.

15. Orthopädietechnischer Schaft nach einem der voranstehenden Ansprüche, **dadurch gekennzeichnet, dass** in der Schaftwand (2) zumindest ein flexibler Bereich (43) ausgebildet ist, der mit einem Versteifungselement (40) gekoppelt ist.

16. Verfahren zur Einstellung eines orthopädietechnischen Schaftes zur Anlage an und Aufnahme von einem Gliedmaßenstumpf oder einer Gliedmaße, der Schaft weist zumindest eine Schaftwand (2), eine proximalen Einstiegsöffnung, ein distales Ende und zumindest eine Befestigungseinrichtung, die zur Festlegung zumindest einer distalen orthopädietechnischen Komponente an dem Schaft angeordnet ist, auf, **dadurch gekennzeichnet, dass** die Steifigkeit der Schaftwand (2) über zumindest eine Verstelleinrichtung (30) und ein Versteifungselement (40) zumindest bereichsweise reversibel verändert wird.

17. Verfahren nach Anspruch 16, **dadurch gekennzeichnet, dass** die Steifigkeit durch eine Verlagerung eines Verstellelementes (20), eine Veränderung des Versteifungselementes (40), das an oder in der Schaftwand (2) angeordnet ist und/oder Aktivierung oder Deaktivierung der Verstelleinrichtung (30) verändert wird.

18. Verfahren nach einem der Ansprüche 16 oder 17, **dadurch gekennzeichnet, dass** die Veränderung der Steifigkeit auf der Grundlage von Sensordaten erfolgt, die von zumindest einem Sensor (70) ermittelt und einer Steuerungseinrichtung (60) übermittelt werden, die einen Aktuator (80) der Verstelleinrichtung (30) aktiviert oder deaktiviert.

19. Verfahren nach einem der Ansprüche 16 bis 18, **dadurch gekennzeichnet, dass** die Steifigkeit durch Verdrehen eines Federelementes, Verschieben eines Federelementes, Blockieren eines Federelementes, Verlagern zumindest eines Versteifungselementes (40), Befüllen oder Entleeren eines Fluidvolumens, Veränderung der Viskosität eines Mediums, Erwärmen, Abkühlen, formschlüssiges in Eingriff Bringen zumindest eines Versteifungselementes (40), Verkeilen zumindest eines Versteifungselementes (40) und/oder Verdrehen zumindest eines Versteifungselementes (40) verändert wird.

20. Verfahren nach Anspruch 17, **dadurch gekennzeichnet, dass** die Steifigkeit durch Verdrehen, Verschieben, Spannen, Blockieren und/oder Aktivieren des Verstellelementes (20) verändert wird.

21. Verfahren nach einem der Ansprüche 16 bis 20, **dadurch gekennzeichnet, dass** die Schaftwand (2) Sektoren (25) aufweist, die unabhängig voneinander hinsichtlich ihrer Steifigkeit verändert werden.

22. Verfahren nach einem der Ansprüche 18 bis 21, **dadurch gekennzeichnet, dass** die Veränderung der Steifigkeit während der Benutzung des orthopädietechnischen Schaftes (1) automatisch, insbesondere in Echtzeit und/oder in Abhängigkeit von der Bewegungssituation erfolgt.

## Claims

1. An orthopedic socket for bearing on and for receiving a limb stump or a limb, having
- at least one socket wall (2),
- a proximal access opening (3),
- a distal end (4), and
- at least one fastening device (5) which is arranged for fastening at least one distal orthopedic component (6) to the socket (1), **characterized in that** at least one stiffening element (40) is arranged or formed on or in the socket wall (2), which stiffening element (40) is assigned an adjustment device (30) and by means of which a stiffness of the socket wall (2) is reversibly variable at least in certain regions.

2. The orthopedic socket as claimed in claim 1, **characterized in that** the stiffening element (40) is mounted displaceably in or on the socket wall (2).

3. The orthopedic socket as claimed in claim 1 or 2, **characterized in that** the adjustment device (30) is arranged on or in the socket wall (2).

4. The orthopedic socket as claimed in one of the preceding claims, **characterized in that** the stiffening element (40) is coupled to the adjustment device (30) via an adjustment element (20), and the adjustment element (20) is designed as a gear wheel, roller, crank, lever, tension means, rotary element, piston, thrust means or valve.

5. The orthopedic socket as claimed in one of the preceding claims, **characterized in that** the adjustment device (30) is designed as an electromagnet, pump, electric motor, heater, cooling element, piezoelectric element, electroactive polymer, force accumulator or manually operable device.

6. The orthopedic socket as claimed in one of the preceding claims, **characterized in that** the stiffening element (40) is designed as a fluid volume, shape memory element, corrugated body, accordion element, toothed body, spring, foam body, phase change material, folding element, damper or electro-adhesion element.

7. The orthopedic socket as claimed in one of the preceding claims, **characterized in that** the adjustment device (30) has an actuator (80) which can be activated and deactivated via a control device (60) assigned to it, which control device (60) is coupled to at least one sensor (70) or switch and activates or deactivates the actuator on the basis of sensor data or a switch operation.

8. The orthopedic socket as claimed in claim 7, **characterized in that** the at least one sensor (70) is designed as a temperature sensor, acceleration sensor, IMU, spatial position sensor, inclination sensor, force sensor, optical sensor, conductor electrode, pressure sensor, DMS or resistance sensor.

9. The orthopedic socket as claimed in claim 7 or 8, **characterized in that** the control device (60) is assigned a memory (61), a data processing device (62) and/or a communication interface (63).

10. The orthopedic socket as claimed in one of the preceding claims, **characterized in that** a receptacle (90) for the adjustment device (30) and/or the stiffening element (40) is arranged in or on the socket wall (2).

11. The orthopedic socket as claimed in one of the preceding claims, **characterized in that** the stiffening element (40) and the adjustment device (30) or the adjustment element (20), the adjustment device (30) and the stiffening element (40) are combined as a stiffening module.

12. The orthopedic socket as claimed in one of the preceding claims, **characterized in that** sectors (25) with different radial elasticity are formed in a proximal edge region (9) of the socket wall (2).

13. The orthopedic socket as claimed in claim 12, **characterized in that** the sectors (25) have different materials, different material thicknesses, different widths of recesses (24) and/or a different density of recesses (24).

14. The orthopedic socket as claimed in claim 12, **characterized in that** the individual sectors (25) are assigned displaceable socket wall segments (40A, 40M, 40R, 40P) as stiffening elements (40).

15. The orthopedic socket as claimed in one of the preceding claims, **characterized in that** at least one flexible region (43) is formed in the socket wall (2) and is coupled to a stiffening element (40).

16. A method for adjusting an orthopedic socket for bearing on and for receiving a limb stump or a limb, the socket having at least one socket wall (2), a proximal access opening, a distal end, and at least one fastening device which is arranged for fastening at least one distal orthopedic component to the socket, **characterized in that** the stiffness of the socket wall (2) is reversibly changed at least in certain regions via at least one adjustment device (30) and a stiffening element (40).

17. The method as claimed in claim 16, **characterized in that** the stiffness is changed by moving an adjustment element (20), changing the stiffening element (40), which is arranged on or in the socket wall (2), and/or activating or deactivating the adjustment device (30).

18. The method as claimed in either of claims 16 and 17, **characterized in that** the change of stiffness takes place on the basis of sensor data which are determined by at least one sensor (70) and transmitted to a control device (60), which activates or deactivates an actuator (80) of the adjustment device (30).

19. The method as claimed in one of claims 16 to 18, **characterized in that** the stiffness is changed by twisting a spring element, moving a spring element, blocking a spring element, displacing at least one stiffening element (40), filling or emptying a fluid volume, changing the viscosity of a medium, heating, cooling, positively engaging at least one stiffening element (40), wedging at least one stiffening element (40) and/or twisting at least one stiffening element (40).

20. The method as claimed in claim 17, **characterized in that** the stiffness is changed by twisting, shifting, tensioning, blocking and/or activating the adjustment element (20).

21. The method as claimed in one of claims 16 to 20, **characterized in that** the socket wall (2) has sectors (25) which are changed independently of one another in terms of their stiffness.

22. The method as claimed in one of claims 18 to 21, **characterized in that** the change in stiffness during the use of the orthopedic socket (1) takes place automatically, in particular in real time and/or as a function of the movement situation.

## Revendications

1. Emboîture orthopédique destinée à être appliquée contre un moignon de membre ou un membre et à recevoir celui-ci, comprenant
- au moins une paroi d'emboîture (2),
- une ouverture d'entrée proximale (3),
- une extrémité distale (4), et
- au moins un dispositif de fixation (5) qui est disposé sur l'emboîture (1) pour fixer au moins un composant orthopédique distal (6),
**caractérisée en ce que** sur ou dans la paroi d'emboîture (2) est disposé ou formé au moins un élément de rigidification (40) auquel est associé un dispositif de réglage (30) et par lequel une rigidité de la paroi d'emboîture (2) peut être modifiée de manière réversible au moins localement.

2. Emboîture orthopédique selon la revendication 1,
**caractérisée en ce que** l'élément de rigidification (40) est monté dans ou sur la paroi d'emboîture (2) de manière à pouvoir être déplacé.

3. Emboîture orthopédique selon la revendication 1 ou 2,
**caractérisée en ce que** le dispositif de réglage (30) est disposé sur ou dans la paroi d'emboîture (2).

4. Emboîture orthopédique selon l'une des revendications précédentes, **caractérisée en ce que** l'élément de rigidification (40) est couplé au dispositif de réglage (30) par l'intermédiaire d'un élément de réglage (20), et l'élément de réglage (20) est conçu comme une roue dentée, un rouleau, une manivelle, un levier, un moyen de traction, un élément rotatif, un piston, un moyen de poussée ou une valve.

5. Emboîture orthopédique selon l'une des revendications précédentes, **caractérisée en ce que** le dispositif de réglage (30) est conçu comme un électroaimant, une pompe, un moteur électrique, un chauffage, un élément de refroidissement, un élément piézoélectrique, un polymère électro-actif, un accumulateur de force ou un dispositif à commande manuelle.

6. Emboîture orthopédique selon l'une des revendications précédentes, **caractérisée en ce que** l'élément de rigidification (40) est conçu comme un volume de fluide, un élément à mémoire de forme, un corps ondulé, un élément en accordéon, un corps denté, un ressort, un corps en mousse, un matériau à changement de phase, un élément de rabattement, un amortisseur ou un élément électro-adhésif.

7. Emboîture orthopédique selon l'une des revendications précédentes, **caractérisée en ce que** le dispositif de réglage (30) comprend un actionneur (80) susceptible d'être activé et désactivé par un dispositif de commande (60) qui lui est associé et qui est couplé à au moins un capteur (70) ou à un commutateur et qui active ou désactive l'actionneur en se basant sur des données du capteur ou sur un actionnement du commutateur.

8. Emboîture orthopédique selon la revendication 7,
**caractérisée en ce que** ledit au moins un capteur (70) est conçu comme un capteur de température, un capteur d'accélération, une unité de mesure inertielle, un capteur de position spatiale, un capteur d'inclinaison, un capteur de force, un capteur optique, une électrode de dérivation, un capteur de pression, une jauge de contrainte ou un capteur de résistance.

9. Emboîture orthopédique selon la revendication 7 ou 8,
**caractérisée en ce qu'**une mémoire (61), un dispositif de traitement de données (62) et/ou une interface de communication (63) sont associés au dispositif de commande (60).

10. Emboîture orthopédique selon l'une des revendications précédentes, **caractérisée en ce qu'**un logement (90) pour le dispositif de réglage (30) et/ou pour l'élément de rigidification (40) est disposé dans ou sur la paroi d'emboîture (2).

11. Emboîture orthopédique selon l'une des revendications précédentes, **caractérisée en ce que** l'élément de rigidification (40) et le dispositif de réglage (30), ou l'élément de réglage (20), le dispositif de réglage (30) et l'élément de rigidification (40) sont réunis en un module de rigidification.

12. Emboîture orthopédique selon l'une des revendications précédentes, **caractérisée en ce que** des secteurs (25) présentant une élasticité radiale différente sont formés dans une zone de bord proximale (9) de la paroi d'emboîture (2).

13. Emboîture orthopédique selon la revendication 12,
**caractérisée en ce que** les secteurs (25) présentent des matériaux différents, des épaisseurs de matériau différentes, des évidements (24) de largeur différente et/ou une densité différente d'évidements (24).

14. Emboîture orthopédique selon la revendication 12,
**caractérisée en ce que** des segments de paroi d'emboîture (40A, 40M, 40R, 40P) déplaçables sont associés aux différents secteurs (25) en tant qu'éléments de rigidification (40).

15. Emboîture orthopédique selon l'une des revendications précédentes, **caractérisée en ce qu'**au moins une zone flexible (43) est formée dans la paroi d'emboîture (2), laquelle est couplée à un élément de rigidification (40).

16. Procédé d'ajustement d'une emboîture orthopédique destinée à être appliquée contre un moignon de membre ou un membre et à recevoir celui-ci, l'emboîture présentant au moins une paroi d'emboîture (2), une ouverture d'entrée proximale, une extrémité distale et au moins un dispositif de fixation qui est disposé sur l'emboîture pour fixer au moins un composant orthopédique distal,
**caractérisé en ce que** la rigidité de la paroi d'emboîture (2) est modifiée de manière réversible au moins localement par au moins un dispositif de réglage (30) et un élément de rigidification (40).

17. Procédé selon la revendication 16,
**caractérisé en ce que** la rigidité est modifiée par un déplacement d'un élément de réglage (20), par une modification de l'élément de rigidification (40) qui est disposé sur ou dans la paroi d'emboîture (2) et/ou par une activation ou une désactivation du dispositif de réglage (30).

18. Procédé selon l'une des revendications 16 ou 17,
**caractérisé en ce que** la modification de la rigidité s'effectue sur la base de données de capteur qui sont déterminées par au moins un capteur (70) et transmises à un dispositif de commande (60) qui active ou désactive un actionneur (80) du dispositif de réglage (30).

19. Procédé selon l'une des revendications 16 à 18,
**caractérisé en ce que** la rigidité est modifiée par une rotation d'un élément élastique, une translation d'un élément élastique, un blocage d'un élément élastique, un déplacement d'au moins un élément de rigidification (40), un remplissage ou une évacuation d'un volume de fluide, une modification de la viscosité d'un fluide, un chauffage, un refroidissement, une mise en prise par complémentarité de forme d'au moins un élément de rigidification (40), un calage d'au moins un élément de rigidification (40) et/ou une torsion d'au moins un élément de rigidification (40).

20. Procédé selon la revendication 17,
**caractérisé en ce que** la rigidité est modifiée par une rotation, une translation, une mise sous contrainte, un blocage et/ou une activation de l'élément de réglage (20).

21. Procédé selon l'une des revendications 16 à 20,
**caractérisé en ce que** la paroi d'emboîture (2) présente des secteurs (25) dont la rigidité est modifiée indépendamment les uns des autres.

22. Procédé selon l'une des revendications 18 à 21,
**caractérisé en ce que** la modification de la rigidité s'effectue automatiquement pendant l'utilisation de l'emboîture orthopédique, en particulier en temps réel et/ou en fonction de la situation de mouvement.
